(19) Europäisches Patentamt
European Patent Office
Office européen des brevets

(11)  **EP 1 791 871 B1**

(12)  **EUROPEAN PATENT SPECIFICATION**

(45) Date of publication and mention
of the grant of the patent:
**15.02.2012  Bulletin 2012/07**

(51) Int Cl.:
**C08B 37/00** *(2006.01)*

(21) Application number: **05786099.1**

(22) Date of filing: **12.08.2005**

(86) International application number:
**PCT/US2005/028608**

(87) International publication number:
**WO 2006/026113 (09.03.2006 Gazette 2006/10)**

(54) **PROCESS FOR PREPARING REDUCED ODOR LOW MOLECULAR WEIGHT CATIONIC POLYGALACTOMANNAN**

VERFAHREN ZUR HERSTELLUNG VON NIEDERMOLEKULAREM KATIONISCHEM POLYGALACTOMANNAN MIT VERRINGERTEM GERUCH

PROCEDE POUR PREPARER POLYGALACTOMANNAN CATIONIQUE DE FAIBLE POIDS MOLÉCULAIRE AYANT UNE ODEUR RÉDUITE

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.08.2004  US 605556 P**

(43) Date of publication of application:
**06.06.2007  Bulletin 2007/23**

(73) Proprietor: **HERCULES INCORPORATED**
**Wilmington, DE 19894-0001 (US)**

(72) Inventors:
• **BEJGER, Thomas, P.**
**Odessa, DE 19730-0012 (US)**
• **ERAZO-MAJEWICZ, Paquita**
**Newark, DE 19711 (US)**
• **HOPKINS, Daniel, L.**
**Hockessin, DE 19707 (US)**

• **KOSTAS, John, N.**
**Wilmington, DE 19803 (US)**
• **KUO, Pong-Kuen, P.**
**Hockessin, DE 19707 (US)**
• **MODI, Jashawant, M.**
**Hockessin, DE 19707 (US)**
• **XU, Zu-Feng**
**Newark, DE 19711 (US)**

(74) Representative: **Bülle, Jan**
**Kutzenberger & Wolff**
**Patentanwaltssozietät**
**Theodor-Heuss-Ring 23**
**50668 Köln (DE)**

(56) References cited:
**WO-A-03/095497    US-A1- 2001 051 140**
**US-A1- 2005 227 902**

## Description

The present invention relates to a process for preparing a reduced odor polygalactomannan composition and more particularly to the preparation of a guar gum composition which when dispersed in water is capable of forming a relatively transparent solution and uses of this polygalactomannan composition in personal care, household, industrial and institutional compositions that have no discernible amine odor at acidic, neutral or alkaline pH values.

## BACKGROUND OF THE INVENTION

Low and high molecular weight cationic galactomannan polymers are used as conditioners in personal cleansing products such as shampoos and body washes, which are typically formulated at acidic or neutral pH values. As a result of processing, an amine odor is apparent in samples of some cationic galactomannan polymers such as cationic guars. At acidic and neutral pH values there is no apparent objectionable "fishy" odor, characteristic of amines, such as trimethylamine (TMA). This is expected, since at acidic or neutral pH values, most amines are in the aqueous phase, in the nonvolatile shalt form. Consequently, there has been no critical need identified for a low odor version of low or high molecular weight cationic galactomannan or cationic guar polymers. However, on incorporation of some cationic galactomannan polymers such as cationic guars in personal cleansing products and in household products, such as detergents and fabric conditioners, formulated at alkaline pH values, an unacceptable odor develops that is characteristic of an amine.

Cationic polysaccharides and other polymers have been used widely in personal care, household, industrial, and institutional products to perform a function in the final product, ranging from the use of the polymer as gellants, binders, thickeners, stabilizers, emulsifiers, spreading and deposition aids and carriers for enhancing the rheology, efficacy, deposition, aesthetic and delivery of chemically and physiologically active ingredients in personal care, household, institutional and industrial compositions. Depending on the application, the substrate to which the product is applied can be skin, hair, or textile substrates.

Cationic polysaccharides are used in hair care products to provide conditioning to the hair. In skin care products, these same polymers can provide conditioning effects to the skin. When incorporated into detergent and fabric softening formulations, these same polymers can provide conditioning, softening, anti-abrasion and antistatic characteristics to fabrics.

Wet and dry combability measurements are typical test methods used to measure conditioning performance in shampoo and conditioner applications. Commercial cationic conditioning polymers in the marketplace have been reported to reduce the wet combing force experienced on combing wet hair by 30%-80% relative to the shampoo containing no polymer. The performance of different cationic polymers in these applications varies, however, in achieving a good balance of wet and dry combing force reduction, with good optical clarity in a formulation. EP1501873 A1 addresses this need for a cationic galactomannan polymer with good optical clarity in personal care, household, and fabric cleansing formulations.

A need still exists in the marketplace, for a cationic galactomannan conditioning polymer that has broad surfactant compatibility, which can deliver clear personal care, household formulations, institutional, and industrial formulations with good conditioning performance with no discernible amine odor at alkaline pH values.

A need also exists in the marketplace for a process that removes amine and other undesired low molecular weight components from polygalactomannan compositions.

WO 03/095497 teaches a composition comprising at least one cationic polygalactomannan or a derivative of cationic polygalactomannans having a mean average molecular weight (Mw) of 5,000 - 200,000, having a light transmittance in a 10% aqueous solution of greater than 80% at a light wavelength of 600 nm, a protein content of less than 1.0% by weight of polysaccharide, and a content of aldehyde functionalities of at least 0.01 meq/gram, This composition is disclosed as being useful in personal care and household care products.

## SUMMARY OF THE INVENTION

The present invention provides a process for preparing a reduced odor composition comprising (a) reacting at least one cationic potygalactomannan or cationic derivatized polygalactomannan with at least one reagent that reduces the Mw to less than 200,000 wherein the reaction mass includes water soluble color bodies and water insoluble material, (b) removing water insoluble material, and (c) removing odorous components, including trimethylamine (TMA) and other amines and low molecular weight components, in an aqueous phase by nitrogen sparging, distillation, ion exchange or membrane diafiltration to produce the reduced odor composition which comprises at least one cationic polygalactomannan or a derivative thereof having: (i) a weight average molecular weight (Mw) of 5,000 - 200,000, (ii) a light transmittance in a 10% aqueous solution of greater than 80% at a light wavelength of 600 nm, (iii) a protein content of less than 1.0% by weight of polysaccharide, and (iv) a trimethylamine content of less than 25 ppm in a 10% aqueous solution of the polymer.

**DETAILED DESCRIPTION OF THE INVENTON**

It has been found unexpectedly that a low odor cationic galactomannan polymer compositions can be produced by application of various methods that reduce the amine content, such as trimethylamine content, in aqueous solutions of cationic galactomannan polymers. It has been found, also, that there is a significant redaction in odor in personal care and household products incorporating the low odor cationic galactomannan polymer compositions of the invention at alkaline pH values.

In addition, the polymers produced by the process of this invention may possess other attributes including their ability to deliver clear formulations across a range of surfactant systems and across a range of polymer concentrations, in personal care and household products. The polymers can also deliver conditioning effects with high clarity in personal care products and in other surfactant-based products, such as household products.

In accordance with the invention, the polymer produced by the process of the invention imparts no malodor or discernible amine odor to personal care, household, or other products when formulated at acidic, neutral, or alkaline pH values when the polymer of the invention is incorporated into the formulation at a level of less than 10 wt %, preferably at a level of less than 5 wt %, and more preferably at a level of less than 1 wt %.

The polymers which are produced by the process of the invention include cationic galactomannan polymers or cationic derivatized galactomannan polymers having a weight average molecular weight (Mw) having a lower limit of 5,000 preferably 20,000, more preferably 35,000, and most preferably 50,000. The upper limit of the Mw of these polymers is less than 200,000, preferably 100,000, and more preferably 70,000. Examples of the polygalactomannans are guar, locust bean, honey locus, and flame tree with guar gum being the preferred source of the polygalactomannan. The preferred polygalactomannan starting material used in this invention is guar flour, guar powder, guar flakes, guar gum, or guar splits which has been derivatized with a cationic substituent.

The preferred polymers produced by the process of this invention are cationic polygalactomannan polymers. The amount of cationic functionality on the polygalactomannan can be expressed in terms of moles of substituent. The term "degree of substitution" as used in this invention is equivalent to the molar substitution, the average number of moles of functional groups per anhydro sugar unit in the polygalactomannan gum. The cationic functionality can be present on these polymers at a DS level as low as 0.01, preferably about 0.1, and more preferably 0.2. The DS upper limit is normally about 3.0, preferably about 2.0, and more preferably 1.0. In addition to molar substitution, the cationic charge on the polymers of this invention can be quantified as a charge density. The molar substitution can be converted to a charge density through a variety of methods. The preferred method for calculating charge density of cationic polymers uses a method that specifically quantifies the equivalents of quaternary ammonium groups on the polymer. Starting material having a cationic molar substitution level of 0.18 has been determined to have a charge density of 0.95 mequivalents per gram (meq/g) according to the following equation:

$$\text{Cationic charge density of DS 0.18 cationic guar} = (1000 \times 0.18)/(162.14 + (151.64 \times 0.18)) = 0.95 \text{ meq/g.}$$

Charge density can be measured by any method that quantifies the net positive or negative charge present on a polymer. The charge density can be determined by measurement of the moles of quaternary ammonium groups bound to the polymer backbone using standard NMR techniques of integration. This method was used for determining the charge density for polymers produced by the process of this invention.

The cationic functionality of the polygalactomannan or derivatized polygalactomannan can be added to them by several methods. For example, the starting material can be reacted for a sufficient time and at a sufficient temperature with tertiary amino compound or quaternary ammonium compound containing groups capable of reacting with the reactive hydrogen ions present on the polygalactomannan or derivatized polygalactomannan in order to add the cationic functionality to the starting material. The sufficient time depends on the ingredients in the reaction mass and the temperature under which the reaction is taking place.

The cationizing agent of the present invention is defined as a compound which, by substitution reaction with the hydroxy groups of the polygalactomannan can make the product electrically positive, and there is no limitation to its types. Tertiary amino compounds or various quaternary ammonium compounds containing groups capable of reacting with reactive hydrogen present on the polysaccharide, can be used, such as 2-dialkylaminoethyl chloride and quaternary ammonium compounds such as 3-chloro-2-hydroxypropyltrimethylammonium chloride, and 2,3-epoxy-propyltrimethylammonium chloride. Preferred examples include glycidyltrialkylammonium salts and 3-halo-2-hydroxypropyltrialkylammonium salts such as glycidyltrimethylammonium chloride, glycidyltriethylammonium chloride, gylcidyltripropylammonium chloride, glycidylethyldimethylammonium chloride, glycidyldiethylmethylammonium chloride, and their corresponding bromides and iodides; 3-chloro-2-hydroxypropyltrimethylammonium chloride, 3-chloro-2-hydroxypropyltriethylammonium chloride, 3-chloro-2-hydroxypropyltripropylammonium chloride, 3-chloro-2-hydroxypropylethyldimethylammonium chloride, and their corresponding bromides and iodides; and quaternary ammonium compounds such as halides of imidazoline ring containing compounds.

Other derivatization of the cationic polygalactomannan with nonionic substituents, i.e., hydroxyalkyl wherein the alkyl represents a straight or branched hydrocarbon moiety having 1 to 6 carbon atoms (e.g., hydroxyethyl, hydroxypropyl, hydroxybutyl) or anionic substituents, such as carboxymethyl groups are optional. These optional substituents are linked to the polygalactomannan molecule by the reaction of the polygalactomannan molecule with reagents such as (1) alkylene oxides (e.g., ethylene oxide, propylene oxide, butylene oxide) to obtain hydroxyethyl groups, hydroxypropyl groups, or hydroxybutyl groups, or with (2) chloromethyl acetic acid to obtain a carboxymethyl group on the polygalactomannan. This reaction can take place when the polygalactomannan is in the "split", "flour" or any other physical form. The process for preparing derivatized polygalactomannan is well known in the art.

In accordance with this invention, the cationic polygalactomannan or cationic derivatized polygalactomannan composition not only has a reduced viscosity and low weight-average molecular weight (Mw) but also has a percent light transmittance in a 10 % aqueous solution of greater than 80 % at a wave length of 600 nm, preferably greater than 90 %, and more preferably greater than 95 %.

In accordance with this invention, the cationic polygalactomannan or cationic derivatized polygalactomannan composition has a trimethylamine content in a 10 % aqueous solution of less than 25 ppm, preferably less than 7 ppm, and most preferably less than 5 ppm when measured by any method known to those skilled in the art. Examples of methods used to measure trimethylamine include gas chromatography (GC), mass spectrometry, solid phase extraction methods using fiber adsorbents, and combinations thereof.

In accordance with this invention, the low molecular weight polygalactomannan has low protein contents. While conventional polygalactomannan gum may have about 3 % protein content, as measured by quantification of percent nitrogen or by use of colorimetric techniques (M.M. Bradford, Anal. Biochem., 1976, 72, 248-254), the polygalactomannan compositions of this invention have a protein content of less than 1 % as measured by the Bradford method, and preferably less than 0.5 %.

Borate salts are commonly used to cross-link guar prior to derivatization. These borate salts generally remain strongly bound to the cationic polygalactomannan product even after water washing. The process steps used to reduce the trimethylamine content of the cationic polygalactomannan polymers also readily removes borate salts from the polymer, producing a higher purity cationic polygalactomannan. In the present invention, the boron content of the polygalactomannan is less than 50 ppm; preferably less than 30 ppm, and more preferably less than 10 ppm per gram of polygalactomannan.

In accordance with the present invention, the molecular weight of polygalactomannans can be reduced as set forth in step a above, by several different methods, such as (1) by biochemical methods wherein polysaccharide hydrolytic enzymes, bacteria, or fungi are used directly, (2) chemical method using (a) acid (b) alkali, or (c) through the use of oxidative agents, i.e., hydrogen peroxide, (3) physical methods using high speed agitation and shearing machines, (4) thermal methods, or (5) depending on necessity, a suitable purification method can be used to make the molecular weight fall within a certain range. Examples of the purification methods that can be used are filtration using a filter-aid, ultrafiltration, reverse osmosis membrane, selective density centrifugation, and chromatography.

In accordance with this invention, an oxidative reagent either alone or in combination with other reagents, including biochemical reagents, is used to reduce molecular weight or introduce oxidized functional groups. In order to achieve optimum results, it is necessary to include the oxidative reagent in the process either completely or alternately with other reagents.

Oxidative agents include any reagent that incorporates oxygen atoms into the polymer structure. Some oxidizing reagents can also act to reduce the molecular weight of the polymer. Examples of these dual function oxidizing agents are peroxides, peracids, persulfates, permanganates, perchlorates, hypochlorite, and oxygen. Examples of biochemical oxidative agents that do not reduce molecular weight but introduce aldehyde functionality are oxidases. Specific examples of oxidases useful in this invention are galactose oxidase, and other biochemical oxidizing agents known to those skilled in the art.

A generalized preferred process for producing the cationic polygalactomannan or derivative of the cationic polygalactomannan composition is as follows:

> (a) reacting a small portion of the cationic polygalactomannan or derivative with an oxidizing reagent or a combination of a hydrolytic reagent and an oxidizing reagent in the presence of water for a sufficient time to reduce the viscosity and molecular weight of the polymer;
> (b) adding additional quantities of the polymer and oxidizing reagent making multiple additions of the polymer and oxidizing agent depending on the desired results and the reaction parameters;
> (c) terminating the reaction and recovering a fluid aqueous dispersion of the composition that contains water soluble color bodies, and water insoluble material, and water at a concentration of about 50 to 95% by weight of the total composition;

(d) removing water insoluble material from the aqueous dispersion to produce a clarified, aqueous solution of the composition of this invention. Conventional means are used for removing the water insoluble materials, such as centrifugation and filtration methods; and

(e) removing of odorous components, including trimethylamine (TMA) and other amines and low molecular weight components in the aqueous phase to produce the cationic polygalactomannan composition of the invention.

Optionally, this process can include an additional step to remove the water soluble color bodies to produce a colorless, clarified aqueous solution of the composition of this invention. Examples of reagents and materials that can be used to remove the color bodies include sodium bisulfite, sodium meta bisulfite, sodium hypochlorite, sodium chlorite, activated carbon, and molecular sieves.

When the combination of the hydrolytic reagent and an oxidizing reagent is used in this invention, the oxidizing reagent will be used in step (b) and the hydrolytic reagent will be used in step (a). This alternating of reagents can be used throughout the process. In another embodiment, all of the hydrolytic reagent and polymer are added batchwise to the reaction vessel and the reaction is allowed to continue to the desired viscosity. If the hydrolytic reagent is an enzyme, it is then deactivated by heat at the end of the reaction. Thereafter, the reaction mass is clarified to a clear solution by conventional processes. An oxidizing reagent is added to the clarified solution and reacted to the desired viscosity and molecular weight for the final product.

Alternatively, the reaction can be performed in a batch process with one addition of reagent (either dual function or combination of hydrolytic reagent and oxidizing reagent) at the beginning of the reaction, with a content of polygalactomannan solids that allows for good mixing using standard stirring equipment. In this batch process, when a combination of reagents are used, the oxidizing reagent can also be added at the beginning with the polymer and the hydrolytic reagent can be added at a later predetermined time in the process in order to achieve the desired results. The neutralization acid used to maintain the reaction in the desired pH range can be any acid, including hydrochloric acid, adipic acid, succinic acid, fumaric acid, malic acid etc.

Alternatively, the reaction with the oxidizing reagent can be conducted in a high-solids state without added water, or in the presence of low levels of water to give a wetted solid rather than an aqueous dispersion at the end of the reaction with the oxidizing agent. In this case, the wetted solid is then mixed with sufficient water to produce a fluid aqueous dispersion for removal of the water insoluble material.

In accordance with the invention, the methods useful in the process of removal of odorous components of the cationic galactomannan polymers are nitrogen sparging, distillation, ion exchange, and membrane diafiltration or combinations thereof. Nitrogen sparging can be done at atmospheric pressure or with the aid of vacuum. Distillation in general could be employed or in this case, where the odiforous components are at such low levels (<100 ppm), extractive distillation, using water as the extractive solvent, would be more effective. Alternatively, polystyrene-based Ion exchange resins could be similarity used to "scavenge" either acidic or basic compounds. Membranes could also be employed to remove low molecular weight impurities, regardless of the chemical characteristics. For instance, a nanofiltration membrane could be used to diafilter the low molecular weight cationic galactomannan polymer. Diafiltration is the process of washing low molecular weight compounds through the membrane with added water. The components that are washed through the membrane and those retained are dependant on the pore size of the membrane. In this diafiltration process, the level of removal of impurities increases with the volume of wash water that is employed. The membrane can be housed in a number of configurations, including hollow fiber, spiral wound, or plate and frame.

The reduced odor polygalactomannan composition prepared by the process of the present invention can be incorporated in functional systems such as personal care products, household care products, and pet care products. Other functional systems include industrial and institutional products, such as hand and body sanitizing products such as liquid soaps, can also be used. The above mentioned functional systems can optionally contain at least one other active personal care, household care, or pet care ingredient, respectively. In certain systems such as hair detangler liquids, gels, or sprays, the polygalactomannan itself can act as the active ingredient because of its affinity for the skin and hair. The functional systems can be oil-in-water or water-in-oil emulsions or solutions or slurries.

In accordance with the invention, examples of personal care products that may be incorporated into the polymer composition include cleansing and conditioning products such as two-in-one shampoos, three-in-one shampoos, shampoos, conditioners, shower gels, liquid soaps, bodywash formulas, styling products, shave gels/creams, body cleansers, and bar soaps.

In accordance with the present invention, the personal care active ingredient must provide some benefit to the user's body. Personal care products includes hair care, skin care, sun care, and oral care products. Examples of substances that may suitably be included in the personal care products according to the present invention are as follows:

1) Perfumes, which give rise to an olfactory response in the form of a fragrance and deodorant perfumes which in addition to providing a fragrance response can also reduce body malodor;

2) Skin coolants, such as menthol, menthyl acetate, menthyl pyrrolidone carboxylate N-ethyl-p-menthane-3-carboxamide and other derivatives of menthol, which give rise to a tactile response in the form of a cooling sensation on the skin;

3) Emollients, such as isopropylmyristate, silicone materials, mineral oils and vegetable oils which give rise to a tactile response in the form of an increase in skin lubricity;

4) Deodorants other than perfumes, whose function is to reduce the level of or eliminate micro flora at the skin surface, especially those responsible for the development of body malodor. Precursors of deodorants other than perfume can also be used;

5) Antiperspirant actives, whose function is to reduce or eliminate the appearance of perspiration at the skin surface;

6) Moisturizing agents, that keeps the skin moist by either adding moisture or preventing from evaporating from the skin;

7) Cleansing agents, that removes dirt and oil from the skin;

8) Sunscreen active ingredients, that protect the skin and hair from UV and other harmful light rays from the sun. In accordance with this invention a therapeutically effective amount will normally be from 0.01 to 10% by weight, preferable 0.1 to 5% by weight of the composition;

9) Hair treatment agents, that conditions the hair, cleans the hair, detangles hair, acts as styling agent, volumizing and gloss agents, anti-dandruff agent, hair growth promoters, hair dyes and pigments, hair perfumes, hair relaxer, hair bleaching agent, hair moisturizer, hair oil treatment agent, and antifrizzing agent;

10) Oral care agents, such as dentifrices and mouth washes, that clean, whiten, deodorize and protect the teeth and gum;

11) Denture adhesives that provide adhesion properties to dentures;

12) Shaving products, such as creams, gels and lotions and razor blade lubricating strips;

13) Tissue paper products, such as moisturizing or cleansing tissues;

14) Beauty aids, such as foundation powders, lipsticks, and eye care;

15) Textile products, such as moisturizing or cleansing wipes; and

16) Nail care products.

In accordance with the present invention, the household care and pet care active ingredient must provide some benefit to the user or pet. Examples of household and pet care products include dish detergents, fabric softeners, antistatic products, pet shampoo, deodorizing spray, and insect repellant products. Examples of active substances that may suitably be included according to the present invention are as follows:

1) Perfumes, which give rise to an olfactory response in the form of a fragrance and deodorant perfumes which in addition to providing a fragrance response can also reduce odor;

2) Insect repellent agent whose function is to keep insects from a particular area or attacking skin;

3) Bubble generating agent, such as surfactants which generates foam or lather;

4) Pet deodorizer such as pyrethrins which reduces pet odor;

5) Pet shampoo agents and actives, whose function is to remove dirt, foreign material and germs from the skin and hair surfaces;

6) Industrial grade bar, shower gel, and liquid soap actives that remove germs, dirt, grease and oil from skin, sanitizes skin, and conditions the skin;

7) All purpose cleaning agents, that remove dirt, oil, grease, germs from the surface in areas such as kitchens, bathroom, public facilities;

8) Disinfecting ingredients that kill or prevent growth of germs in a house or public facility;

9) Rug and Upholstery cleaning actives which lift and remove dirt and foreign particles from the surfaces and also deliver softening and perfumes;

10) Laundry softener actives which reduces static and makes fabric feel softer,

11) Laundry detergent ingredients which remove dirt oil, grease, stains and kills germs and inhibit redeposition of substances;

12) Dishwashing detergents which remove stains, food, germs;

13) Toilet bowl cleaning agents which removes stains, kills germs, and deodorizes;

14) Laundry prespotter actives which helps in removing stains from clothes;

15) Fabric sizing agent which enhances appearance of the fabric;

17) Vehicle cleaning actives which removes dirt, grease, etc. from vehicles and equipment;

18) Lubricating agent which reduces friction between parts; and

19) Textile agents, such as dusting collection agents and cleaning agents.

The above list of personal care and household active ingredients are only examples and are not a complete lists of active ingredients that can be used. Other ingredients that are used in these types of products are well known in the industry. In addition to the above ingredients conventionally used, the reduced oder composition can optionally also include ingredients such as a colorant, preservative, antioxidant, nutritional supplements, alpha or beta hydroxy acid, activity enhancer, emulsifiers, functional polymers, viscosifying agents (such as NaCl, NH4Cl, KCl, Na$_2$SO$_4$, fatty alcohols, fatty acid esters, fatty acid amides, fatty alcohol polyethyleneglycol ethers, sorbitol polyethyleneglycol ethers, cocamide monoethanolamide, cocamide diethanolamide, cocamidopropyl betaine, clays, silicas, cellulosic polymers, and xanthan), suspending agents (such as clays, silica, and xanthan), alcohols having 1-6 carbons, fats or fatty compounds, antimicrobial compound, zinc pyrithione, silicone material, hydrocarbon polymer, emollients, oils, surfactants, medicaments, flavors, fragrances, rejuvenating reagents, and mixtures thereof.

In accordance with the present invention, examples of functional polymers that can be used in blends with the reduced odor cationic polygalactomannan or derivatives thereof include water-soluble polymers such as anionic, hydrophobically-modified, and amphoteric acrylic acid copolymers, vinylpyrrolidone homopolymers; cationic, hydrophobically-modified, and amphoteric vinylpyrrolidone copolymers; nonionic, cationic, anionic, and amphoteric cellulosic polymers such as hydroxyethylcellulose, hydroxypropylcellulose, carboxymethylcellulose, hydroxypropytmethylcellulose, cationic hydroxyethylcellulose, cationic carboxymethylhydroxyethylcellulose, and cationic hydroxypropylcellulose; acrylamide homopolymers and cationic, amphoteric, and hydrophobically-modified acrylamide copolymers, polyethylene glycol polymers and copolymers, hydrophobically-modified polyethers, hydrophobically-modified polyetheracetals, hydrophobically-modified polyols and polyetherurethanes and other polymers referred to as associative polymers, hydrophobically-modified cellulosic polymers, polyethyleneoxide-propylene oxide copolymers, and nonionic, anionic, hydrophobically-modified, amphoteric, and cationic polysaccharides such as xanthan, chitosan, carboxymethyl guar, alginates, hydroxypropyl guar, carboxymethyl guar hydroxypropyltrimethylammonium chloride, guar hydroxypropyltrimethylammonium chloride, hydroxypropyl guar hydroxypropyltrimethylammonium chloride.

In accordance with the invention, the silicone materials which can be used are, in particular, polyorganosiloxanes that are insoluble in the composition and can be in the form of polymers, oligomers, oils, waxes, resins, or gums.

The organopolysiloxanes are defined in greater detail in Walter Noll's "Chemistry and Technology of Silicones" (1968) Academic Press. They can be volatile or non volatile.

If volatile, the silicones are more particularly chosen from those having a boiling point of between 60° C. and 260° C., and even more particularly from:

(i) cyclic silicones containing from 3 to 7 and preferably from 4 to 5 silicon atoms. These are, for example, octamethylcyclotetrasiloxane sold in particular under the name "Volatile Silicone 7207" by Union Carbide or "Silbione 70045 V 2" by Rhone Poulenc, decamethyl cyclopentasiloxane sold under the name " Volatile Silicone 7158" by Union Carbide, and "Silbione 70045 V 5" by Rhone Poulenc, and mixtures thereof. Mention may also be made of mixtures of cyclic silicones with organosilicone compounds, such as the mixture of octamethylcyclotetrasiloxane and tetratrimethylsilylpentaerythritol (50/50) and the mixture of octamethylcyclotetrasiloxane and oxy I,I' bis(2,2,2',2',3,3'hexatrimethylsilyloxy) neopentane;

(ii) linear volatile silicones having 2 to 9 silicon atoms and having a viscosity of less than or equal to 5x10-6 m2/s at 25° C. An example is decamethyltetrasiloxane sold in particular under the name "SH 200" by Toray Silicone Company. Silicones belonging to this category are also described in the article published in Cosmetics and Toiletries, Vol. 91, Jan. 76, pp. 27 32, Todd & Byers "Volatile Silicone Fluids for Cosmetics".

Non volatile silicones, and more particularly polyarylsiloxanes, polyalkylsiloxanes, polyalkylarylsiloxanes, silicone gums and resins, polyorganosiloxanes modified with organofunctional groups, and mixtures thereof, are preferably used.

In accordance with the invention, the silicone polymers and resins which can be used are, in particular, polydiorganosiloxanes having high number-average molecular weights of between 200,000 and 1,000,000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Examples of these silicone polymers and resins are as follows:

Polydimethylsiloxane,
polydimethylsiloxanes/methylvinylsiloxane gums,
polydimethylsiloxane/diphenylmethylsiloxane,
polydimethylsiloxane/phenylmethylsiloxane, and
polydimethylsiloxane/diphenylsiloxanemethylvinylsiloxane.

Products which can be used more particularly in accordance with the invention are mixtures such as:

(a) mixtures formed from a polydimethylsiloxane hydroxylated at the end of the chain (referred to as dimethiconol according to the nomenclature in the CTFA dictionary) and from a cyclic polydimethylsiloxane (referred to as cyclomethicone according to the nomenclature in the CTFA dictionary), such as the product Q2 1401 sold by the Dow Coming Company;

(b) mixtures formed from a polydimethylsiloxane gum with a cyclic silicone, such as the product SF 1214 Silicone Fluid from the company General Electric Company; this product is an SF 30 gum corresponding to a dimethicone, having a number average molecular weight of 500,000, dissolved in SF 1202 Silicone Fluid oil corresponding to decamethylcyclopentasiloxane; and

(c) mixtures formed of two PDMSs of different viscosities, and more particularly of a PDMS gum and a PDMS oil, such as the product SF 1236 from the General Electric Company. The product SF 1236 is a mixture of a gum SE 30 defined above, having a viscosity of 20 m2/s, and an oil SF 96, with a viscosity of 5X10-6 m2/s. This product preferably contains 15% SE 30 gum and 85% SF 96 oil.

These silicone materials in personal care and household products function as conditioning agents for hair, skin, and textile surfaces. Other types of conditioning agents include hydrocarbon oils, such as mineral oil and fatty acid ester of glycerol, and panthenol and its derivatives, such as panthenyl ethyl ether, panthenyl hydroxypropyl steardimonium chloride, and pantothenic acid.

For a more detailed understanding of the invention, reference can be made to the following examples which are intended as further illustration of the invention and are not to be construed in a limiting sense. All parts and percentages are by weight unless stated otherwise.

**EXAMPLES**

In the following Examples the level of trimethylamine (TMA) was measured using two different methods. The limit of detection for Method 1 was ascertained to be 7 ppm. A second method, Method 2, was developed to measure lower levels of TMA in the low molecular weight cationic guar product "PPM" means parts per million. "PPB" means parts per billion. All parts and percentages are by weight unless otherwise specified.

**Standard Methods and Procedures**

Method 1 and / or Method 2 were used for analysis of the TMA level in all Examples shown in Tables 2, 3, and 4.

Method 1: Trimethylamine in Low Molecular Weight Cationic Guar by Headspace GC

The samples were prepared by weighing ~ 0.5 g of the low molecular weight cationic guar solution into the headspace vial and then adding 5 ml of tris buffer at pH -8.5. The vials were equilibrated at - 40° C for 15 minutes prior to injection into the gas chromatography (GC) inlet and quantified using flame ionization detection (FID). Calibration was determined with external standards in equal volumes of the buffer. The detection limit was - 7 ppm.

Method 2:Trimethylamine in Low Molecular Weight Cationic Guar by Headspace SPME/GC/FID

This method was used to determine trimethylamine (TMA) in cationic guar polymer solutions. The sample was adjusted to pH 8.5 with buffer in a headspace vial and TMA was extracted with a solid phase microextraction (SPME) fiber. TMA was desorbed from the fiber in a gas chromatography (GC) inlet and quantitated using flame ionization detection (FID). Calibration was determined with external standards. The result was reported as ug/g (ppm) to two significant figures.

Apparatus

(1) Gas Chromatograph, Agilent 6890A, equipped with a flame ionization detector (FID), a split/splitless injector, a 0.75 mm ID SPME liner (Restek cat. no. 21110), a Merlin Microseal GC inlet seal (Aldrich cat. no. 22581-U), a Gerstel MPS-2 SPME autosampler and an Agilent DB-Wax column, 30 m x 0.53 mm x 1.0 $\mu$m (VWR cat no. 21512-352).

(2) SPME fiber assembly, 50/30 $\mu$m DVB/Carboxen/PDMS StableFlex for auto holder, gray (Aldrich cat. no. 57329-U).

Reagents

(1) Trimethylamine hydrochloride (TMA*HCL), reagent grade (98%), CAS# 593-81-7 (Aldrich cat. no. T72761).

(2) Water, reagent grade, CAS #7732-185 (VWR cat. no, 365-4).

(3) TRIS buffer (VWR cat. no. JTX171-5).

(4) o-Phosphoric acid, 85% (VWR cat. no. EM-552-3).

(5) TRIS buffer solution, pH 8.5 was prepared by adding 10.8 g of TRIS buffer to 500 mL reagent grade water and then titrated to pH 8.5 with concentrated phosphoric acid.

Calibration

(1) A stock calibration solution was prepared by weighing about 40 mg TMA*HCL in a 25-mL volumetric flask and recording the weight to the nearest 0.0001 g. The solution was brought to volume with reagent grade water and mix well. Use Equation (1) to calculate the concentration of TMA in the solution as $\mu$g/mL.

(2) A diluted stock calibration solution was prepared by pipetting 0.25 mL of the stock solution into a 25-mL volumetric flask and brought to volume with reagent grade water and mixed well.

(3) The following amounts of diluted stock solution were pipetted into 10-mL volumetric flasks and brought to volume with reagent grade water to prepare the working standards. Equation (2) was used to calculate the exact level of TMA in each standard.

| Standard # | Diluted Stock, mL |
| --- | --- |
| 1 | 0.5 |
| 2 | 1.0 |
| 3 | 1.5 |
| 4 | 2.0 |

(4) 1 mL of pH 8.5 TRIS buffer was pipetted into a 10-mL headspace vial and 1 mL of Standard #1 was pipetted into the vial. A headspace cap was crimped onto the vial immediately.

(5) Step (4) was repeated for the three remaining Standards.

(6) The four Standards were analyzed using the parameters listed for the MPS-2 auto sampler and the Agilent 6890A GC/FID.

(7) Excel software was used to calculate the slope and y-intercept for the four Standards with the concentrations as the x values and the areas as the y values. If ChemStation is available, it can be used instead of Excel to determine the calibration curve.

Procedure

(1) The volume of sample specified in the following Table 1 was pipetted into a 10-mL volumetric flask. The weigh was recorded to the nearest 0.0001 g. The sample was brought to volume with reagent grade water and mixed well. 1 mL of the sample solution was pipetted into a 10-mL headspace vial.

Table 1

| Expected ug/g | mL sample | Dilution Factor |
| --- | --- | --- |
| 1 to 4 | 5 | 2 |
| 4 to 10 | 2 | 5 |
| 10 to 20 | 1 | 10 |
| 20 to 50 | 0.4 | 25 |
| 50 to 100 | 0.2 | 50 |

(2) 1 mL pH 8.5 TRIS buffer solution was pipetted into the vial and a headspace cap was crimped onto the vial immediately. The vial was gently swirled to mix.

(3) The sample was analyzed using the parameters listed for the MPS-2 auto sampler and the Agilent 6890A GC/FID.

(4) The TMA level was calculated using Equation (3) or using ChemStation according to the parameters listed for Agilent ChemStation. If the area of the sample is outside the calibration range, dilute the sample appropriately in water and rerun.

Gerstel MPS-2 Parameters

Cycle SPME

Syringe Fiber

Pre-incubation Time00:05:00 hrmin:sec

Incubation Temp. 35.0°C

Agitator Speed not used

Agitator On Time 0s

Agitator Off Time 0s

Vial Penetration 22.0 mm

Extraction Time 00:10:00 hr:min:sec

Desorb To GC Injector

Injection Penetration 54.0 mm

Desorb Time 00:05:00 hr:min:sec

Fiber Bakeout 00:00:00 hr:min:sec

GC Runtime 00:20:00 hr:min:sec

Agilent 6890A Gas Chromatograph Operating Conditions

Injection Manual

Oven Initial Temperature 50°C

Initial Time 0.00 min

Rate 20.00 °C /min

Final Temperature 230°C

Equilibration Time 0.50 min

Inlet (Split/Splitters) Mode Splitless

Temperature 270°C
Pressure (4.00 psi) 5.8 kPa
Gas Type Helium

Detector (FID) Temperature 250°C

Makeup Gas Helium
Combined Flow 20.0 mL/min

Agilent ChemStation Parameters

Calibration Table Calculate External Standard Percent
Based On Peak Area
Curve Type Linear

Origin Ignore
Weight Equal
Standard Amount ng/μL (μg/mL)

Sequence Table Sample Amount g

Multiplier .01
Dilution Factor listed in Table 1 in the Procedure Section

Calculations

$$\frac{\text{Wt Std} \times P \times 0.617 \times 0.01 \times 1{,}000{,}000}{25} = \text{TMA in stock, μg/mL} \qquad \text{Eq (1)}$$

where:

Wt Std = weight of TMA*HCl, g
P = purity of TMA*HCl, %
0.617 = ratio of TMA MW to TMA*HCl MW
0.01 = conversion factor for %
1,000,000 = conversion factor for μg to g
25 = dilution volume, mL

$$\frac{V\,ds \times C\,tma}{1000} = \text{TMA in standard, μg/mL} \qquad \text{Eq (2)}$$

where:

V ds = volume of diluted stock solution, mL
C tma = TMA in stock, μg/Ml

$$\frac{(A\,spl - b) \times DF}{m \times Wt\,Spl} = \text{TMA, μg/g (ppm)} \qquad \text{Eq (3)}$$

where:

A spl = area of TMA peak for sample
b = y-intercept for area vs. TMA concentration
DF = dilution factor, from Table 1 iri the Procedure section

m = slope for area vs. TMA concentration
Wt Spl = weight of sample solution, g

**Precursor EXAMPLES 1 and 2**

The precursors for the present invention were prepared using the following procedure.

The following ingredients were added to a 3800 litre (1000 gallon) glass lined reactor. The water, peroxide, and malic acid were added to the reactor with stirring. The cationic guar and sodium hydroxide were added to this mixture. The mixture was heated to a temperature of 85° C, until the viscosity of a sample of the reaction mixture reached the desired viscosity. At this time, the sodium metabisulfite was added. Adipic acid and Phenoxetol® product were added to the reaction product, and the reaction product was removed from the reactor.

Water 1500 litre (390 gal) water (initial) + 1300 litre (350 gal) water (fed w/ slurry)
CatGuar 360 kg (800 lbs) (88 to 91 % TS)
Peroxide: 20.5kg (45.2 lbs) (35%)
NAOH: 16 to 18kg (35 to 40 lbs) (25%)
Malic Acid: 9.5 to 11kg (20.8 to 25 lbs) added during slurry add'n
Sodium metabisulfite (SMBS): 7.5 to 10.5kg (17 to 23 lbs) + optional addition
Adipic Acid: 2 kg (4.5 lbs)@ packout
Phenoxetol: 16.7 kg (36.8 lbs)

The reaction slurry was then subjected to a filtration step to remove water insoluble material using a diaphragm filter press.

As shown in Table 2, the level of trimethylamine (TMA) in the product of Example 1 was measured as 67 ppm using Method 1. The level of TMA in the product of Example 2 was measured as 64 ppm. An amount of 6 kg (13.5 lbs) of Nipasept® sodium was added to the product, with an aliquot of malic acid, to return the pH value to neutral.

The procedures that were used in the Examples to reduce or remove odorous components or low molecular weight components from the product are described in Examples 3 -13.

**EXAMPLES 3-4 and Comparative EXAMPLES 5-6**

Adsorbents were used to remove odor components from the Precursors of Examples 1 and 2.

Examples 3-6 were prepared by adding -100 grams of low molecular weight cationic guar (LMWCG) to 110 (4 ounce) bottles and then for each treatment between 5-20 grams of adsorbent were added. Prior to treatment with charcoal, the pH of the LMWCG was adjusted to pH 8.5 using aqueous 5 % NaOH. The adsorbent / LMWCG slurries were stirred with magnetic stirrer bars for approximately eight hours at 400 rpm. Then the battles were placed in a rotating shaker for one hour at 30 cycles / minute. The contents of the bottles were allowed to settle and the adsorbent-treated LMWCG solutions were sampled by decanting the liquid from the solid adsorbent. The Dowex G-26 treated LMWCG solution was filtered through a porcelain Buchner funnel. The pH of 28g (one ounce) aliquot was adjusted to pH 8 using aqueous 5 % NaOH. The pH of another aliquot was adjusted to pH 6 using 5 % NaOH.

For the adsorbent treatments shown in Table 2, Examples 3-6, the trimethylamine level was reduced from 67 ppm trimethylamine in the untreated LMWCG solution to 59 ppm in the charcoal treated LMWCG and to <7 ppm in the Dowex G-26 (cationic exchange resin) and zeolite (Type H. ZSM-5) treated LMWCG.

**Comparative EXAMPLE 7**

A continuous column treatment of low molecular weight cationic guar with zeolite was conducted using a 33mm (1.3 inch) internal diameter glass column that was packed with 262 g of Degussa ZSM-5 Type H zeolite 3mm (1/8th inch) extrudate pellets. The 10 wt % aqueous solution of low molecular weight cationic guar product of Example 1 was pumped through the column at a rate of 20 g/min. A total of 1150 g of product was collected having a level of 2.5 ppm TMA as measured by Method 2.

**EXAMPLE 8**

A continuous column treatment of low molecular weight cationic guar solution was conducted using a 33mm (1.3 inch) internal diameter glass column that was packed with 186 g (dry weight) of Rohm and Haas Ambertyst® 15 ion exchange resin beads (0.74 mm (0.029 inch) diameter). A 10 wt % aqueous solution of low molecular weight cationic guar, prepared according to the method in Example 1, was pumped through the column at a rate of 18 g/min. A total of 1000 g of product was collected, having a TMA level of 1.4 ppm, as measured by Method 2.

### EXAMPLES 9 and 10

Diafiltration of low molecular weight cationic guar product prepared according to the procedure in Example 1 through a New Logic VSEP (Vibrating SEParator) with a pore size of 200 Dalton at pressures between 200 and 300 psig, at temperatures between 25 and 45° C. The dilution water is acidic (malic acid) and also contains salt (NaCl). The product retentate collected contained a level of 0.64 ppm (Example 9) and 6.5 ppm trimethylamine (for Example 10), as measured by Method 2.

A reduced level of boron was measured in the product retentate relative to the untreated polymer in Example 1, as measured by diluting the polymer sample with 4 % $HNO_3$ in DI (deionized) water. The diluted samples were analyzed by inductively coupled plasma - atomic-emission spectroscopy.

### EXAMPLE 11

Removal of TMA from low molecular weight cationic guar solution was demonstrated using a Liqui-Cel® hollow-fiber membrane contactor using the following procedure. Liqui-Cel® and Celgard® are registered trademarks of Hoechst Celanese Corp.

The low molecular weight cationic guar solution was heated to 60° C with stirring in a vessel, and the pH was adjusted to 9 with base. The cationic guar solution flowed into the shell-side of the contactor, while maintaining feed pressure at or below 140 kPa (20 psi). Nitrogen gas flowed into the tube-side of the contactor, maintaining the N2 pressure at 140 kPa (20 psi) or less- The cationic guarwas allowed to continuously recirculate back into the heated vessel. The $N_2$ stream was passed through the contactor once and vented. This process was allowed to continue for up to 5 hours. The product was then cooled to 25° C and the pH adjusted to 6 with malic acid.

### EXAMPLE 12

Nitrogen sparging of aqueous solutions of low molecular weight cationic guar reduced the level of TMA in the product as shown in Table 2, Example 12. The aqueous solution of cationic guarwas heated to 60° C in a stirred flask, the pH was adjusted to 8.5, and nitrogen sparging was performed for 2 hours at this temperature. The level of TMA in the product was reduced from 67 ppm to 16 ppm using this process.

### Comparative EXAMPLE 13

It has been found that the level of amine odor and the level of TMA in aqueous solutions of low molecular weight cationic galactomannan polymers are significantly reduced when combined enzyme-peroxide processing is used to reduce the molecular weight of the cationic galactomannan polymer, instead of the peroxide oxidation process. The GC headspace analyses shown in Table 2, Example 13 also demonstrate the reduction in TMA levels In aqueous solutions of cationic galactomannan polymers that have been prepared using enzyme. peroxide combined processing (Example 13) compared with the peroxide process (Example 1).

Table 2. Effect of Treatment on Trimethylamine (TMA) Concentration in Low Molecular Weight Cationic Guar (Polygalactomannan) Solutions (10 weight percent total solids).

| Example | Treatment Description | Adsorbent Concentration (Weight percent) | Boron/ wt% | Wt% Protein | TMA/ppm Method 1 | TMA/ppm Method 2 |
|---|---|---|---|---|---|---|
| Example 1 | None Comparative Control | - | 36 | 0.38 | 87 | |
| Example 2 | None (contol) | - | | | | 64 |
| Example 3 | Dowex G2θ, pH8, Ion exchange resin | 16.5 | | 0.37 | Not detected <7ppm* | |

(continued)

| Example | Treatment Description | Adsorbent Concentration (Weight percent) | Boron/ wt% | Wt% Protein | TMA/ppm Method 1 | TMA/ppm Method 2 |
|---|---|---|---|---|---|---|
| Example 4 | Dowex G26, pH6, Ion exchange resin | 16.5 | | | Not detected <7ppm* | |
| † Example 5 | Zeolite Type H-ZSM-5 | 9.1 | | 0.199 | Not detected <7ppm* | |
| † Example 6 | Charcoal. pH 8.5 | 4.5 | | | 59 | |
| † Example 7 | Degussa ZSM-5 Type-H Zeolite Type 1/8ln. extrudate pellets | Column | | | | 2.5 |
| Example 8 | Ambenyst® 15 0.029 in chameter Rohm and Haas | Column | | | | 1.4 |
| Example 9 | Dialfiltration pH 5.6 | - | <5 | | <7ppm* | 0.64 |
| Example 10 | Diafiltration pH 5.8 | - | | 0.44 | | 6.5 |
| Example 11 | Hollow Fiber Membrane | - | | - | | |
| Example 12 | Nitrogen Sparging, pH9, 2hrs, 60 °C | - | | | 18ppm | |
| † Example 13 | Mannanase enzyme-peroxide degradation | - | | 0.81 | Not detected <7ppm* | |
| * 7 ppm was the detection limit for analytioal Method 1; † Comparative Example | | | | | | |

The analyses shown in Examples 3-13 compared with Example 1 and 2 in Table 2 demonstrate the reduction in the level of trimethylamine (TMA) in aqueous solutions of a cationic guar subjected to these post-treatments.

In accordance with the invention, in a preferred process, the molecular weight reduction step is conducted in aqueous medium to produce a dispersion, and water insoluble solids are removed from the dispersion, and one of the processes shown in the Examples 3 through 12 in Table 2 is applied, to produce a clarified, low odor solution of the galactomannan polymer composition of the invention. Optionally, water soluble color bodies are removed to make a colorless, clarified, low odor, aqueous solution of the cationic galactomannan polymer or derivatized cationic galactomannan polymer. Optionally, the resultant cationic galactomannan polymer or derivatized cationic galactomannan polymer can also be recovered in dry form from solution.

15

**Examples 14 -16**

The reduced perception of odor in personal care compositions containing the low odor cationic polygalactomannan samples of the invention are demonstrated in the following Examples and the results are reported in Tables 3 and 4. The procedure for preparing the shampoo formulations used in Table 3 is described in the following procedure.

PROCEDURE: Conditioning Shampoo

Shampoo formulations were prepared, containing low molecular weight cationic guar of the invention, an untreated cationic guar, or a control shampoo in which water was substituted for the cationic guar solution. The aqueous solutions of cationic guar were adjusted for their total solids content and the water charge was adjusted accordingly. Two samples of each formulation were made, one sample adjusted to pH 5.5-6.0 and one sample adjusted to pH 8.0-8.5.

Shampoo Formulation:

| Examples | 14 | 15 | 16 |
|---|---|---|---|
| Part A | | | |
| Deionized water | 65.56 | 65.56 | 65.56 |
| Hydroxyethylcellulose (HEC) | 1.04 | 1.04 | 1.04 |
| 1.0 % NaOH solution | q.s. | q.s. | q.s. |
| Part B | | | |
| Sodium lauryl sulfate (SLS) | 17.00 | 17.00 | 17.00 |
| Sodium laureth sulfate (SLES) | 13.00 | 13.00 | 13.00 |
| Cocamindopropylbetaine (CAPB) | 2.50 | 2.50 | 2.50 |
| DMDM hydantoin | 0.50 | 0.50 | 0.50 |
| Part C | | | |
| Deionized water | 0.40 | 0.00 | 0.00 |
| Cationic guar[1] | 0.00 | 0.40 | 0.00 |
| Cationic guar[2] | 0.00 | 0.00 | 0.40 |
| | 100.00 | 100.00 | 100.00 |

**1. Polymer of Example 2 AquaCat CG-518, marketed by Hercules Incorporated**
**2. Polymer of Example 10**

Part A -Deionized water was charged to the mixing vessel, HEC was added while mixing, and the mixture was stirred for 10 minutes to disperse. The pH of mixture was adjusted to 8.0-8.5 with 1.0 % NaOH solution. The mixtured was then stirred for 30 minutes, then the pH was re-adjusted to 8.0-8.5, and the mixture was continued stirring for an additional 30 minutes.
Part B - Order of addition to Part A while mixing: SLES, CAPB, Methyl gluceth-20, DMDM hydantoin. Mixed 90 minutes.
Part C - Added AquaCat or water to Parts A and B while mixing, mixed 15 minutes.
Shampoo at pH 5.5-6.0 X33768-76A-1, -76B-1, -76C-1: Adjusted shampoo to pH 5.5-6.0 with 5.0 % Citric acid solution, mixed 15 minutes.
Shampoo at pH 8.0-8.5 X33768-76 A-2. -76 B-2, -76 G2: Adjusted shampoo to pH 8.0-8.5 with 1.0% NaOH solution, mixed 15 minutes.

| Material | Trademark | Supplier |
|---|---|---|
| Hydroxyethylcellulose 96.45% active | Natrosol®250HHR-CS | Hercules Inc. |
| Sodium laureth sulfate | Rhodapex® ES-STD | Rhodia |

(continued)

| Material | Trademark | Supplier |
|---|---|---|
| 27% active (3-EO) | | |
| Cocamidopropylbetaine | Amphosol® CA | Stepan Co. 30% active |
| Methyl gluceth-20 | Glucam® E-20 | Amerchol Corp. 100% |
| DMDM Hydantoin | Glydant® | Lonza Group 100% active |
| Cationic guar solution | AquaCat® CG-518 | Hercules Inc. 10.11 %active |
| cationic guar solution | AquaCat® CG-518 | |

Table 3. Odor Panel Assessment of Shampoo Formulations at Acidic and Alkaline pH Values

| Polymer | Example | DS | Mw | Polymer/ wt% | TMA/ ppm in Polymer | Odor Ranking of Shampoos at pH 5.5 and 8.5 by Panelists 1, 2, and 3 | | | | | |
|---|---|---|---|---|---|---|---|---|---|---|---|
| | | | | | | pH 5.5 Panelist 1 | pH 8.5 Panelist 1 | pH 5.5 Panelist 2 | pH 8.5 Panelist 2 | pH 5.5 Panelist 3 | pH 8.5 Panelist 3 |
| Control - water | 14 | --- | --- | 0 | 0 | None | None | None | None | Slight- not offensive | Moderate soapy– not offensive |
| Example 10 | 15 | 0.2 | 39,900 | 0.4% | 6.5 | None | None | None | None | Moderate not offensive | Moderate soapy– not offensive |
| Example 2 | 16 | 0.2 | 41,800 | 0.4% | 64 | None | Slight fishy | Slight-moderat e amine | Stronger amine | Moderate not offensive | Considerable fishy, offensive |

Odor panelists were asked to open each sample and assess the headspace for odor for 15 seconds. As shown by the results in Table 3, as expected, in shampoos formulated at a pH of 5.5-6.0, only one of the panel members detected a slight amine odor in the shampoo formulated with the untreated polymer of Example 2, which had a measured TMA level of 64 ppm.

In shampoos formulated at a pH of 8.0-8.5, all panel members detected amine, fishy, or offensive odor in shampoos formulated with the untreated polymer of Example 2, which had a measured TMA level of 64 ppm. None of the panel members detected amine, fishy, or offensive odor in the shampoo formulated with the polymer of the invention, Example 10, which had a measured TMA level of 6.5 ppm.

**Examples 17-19**

Bodywash formulations were prepared, containing low molecular weight cationic guar of the invention, an untreated cationic guar, or a control in which water was substituted for the cationic guar solution. The aqueous solutions of cationic guar were adjusted for their total solids content and the water charge was adjusted accordingly. Two samples of each formulation were made, one sample adjusted to pH 5.5-6.0 and one sample adjusted to pH 8.0-8.5. The procedure and bodywash formulation are described below.

PROCEDURE: Body Wash at pH 5.5-6.0

A large batch of the body wash formulation was prepared in which 5.0 % of the water charge was held out in order to allow for the addition of the various cationic guar polymer solutions. Body washes containing 0.40 % active cationic guar were prepared by adding 5.0 ppw (parts per weight) of the aqueous cationic guar solution to 95 ppw of the bodywash stock solution. Three body wash formulations were prepared, containing either untreated low Mw cationic guar of Example 2, the treated cationic guar polymer of the invention, Example 10, or a control body wash in which water was substituted for the cationic guar solution. charge was adjusted accordingly. Two samples of each formulation were made, one sample adjusted to pH 5.5-6.0 and one sample adjusted to pH 8.0-8.5.

Body Wash Stock Solution:

| Part A | |
| --- | --- |
| Deionized water | 47.96 |
| Hydroxyethylcellulose (HEC) | 1.04 |
| 1.0 % NaOH solution | q.s. |
| Part B | |
| Sodium laureth sulfate (SLES) | 42.00 |
| Cocamindopropylbetaine (CAPB) | 3.00 |
| Methyl gluceth-20 | 0.50 |
| DMDM hydantoin | 0.50 |
| | 90.00 |

Part A -Deionized water was charged to the mixing vessel, HEC was added while mixing, and the mixture was stirred 10 minutes to disperse. The pH of mixture was adjusted to 8.0-8.5 with 1.0 % NaOH solution. The mixture was stirred for an additional 30 minutes; then the pH of the mixture was re-adjusted to 8.0-8.5; and the mixture was continued mixing for an additional 30 minutes.
Part B - Order of additions to Part A while mixing: SLES, CAPB, Methyl gluceth-20, DMDM hydantoin. Mixed 90 minutes.

Body Wash at pH 5.5-6.0 Examples 17,18,19

| Stock solution | 95.00 |
| --- | --- |
| Cationic guar solution | 5.00 |
| Deionized water | q.s. |
| 5.0 % Citric acid solution | q.s. |
| | 100.00 |

The cationic guar solutions and/or water were added while mixing the bodywash stock solution and were mixed for 15 minutes. The samples were adjusted to pH 5.5-6.0 with 5.0 % citric acid and mixed another 15 minutes.

| Body Wash at pH 8.0 - 8.5: Examples 17,18,19 | |
| --- | --- |
| Stock solution | 95.00 |
| Cationic guar solution | 5.00 |
| Deionized water | q.s. |
| 1.0 % NaOH solution | q.s |
| | 100.00 |

The cationic guar solutions and/or water were added while mixing to the bodywash stock solution and mixed for 15 minutes. The samples were adjusted to pH 8.0-8.5 with 1.0 % NaOH solution and mixed another 15 minutes.

**Table 4. Odor Panel Assessment of Bodywash Formulations at Acidic and Alkaline pH Values**

| Polymer | Example | DS | Mw | Polymer/wt% | TMA/ppm in Polymer | Odor Ranking of Bodywash at pH5.5 and 8.5 by Panelists 1, 2 and 3 | | | | | |
|---|---|---|---|---|---|---|---|---|---|---|---|
| | | | | | | pH 5.5 Panelist 1 | pH 8.5 Panelist 1 | pH 5.5 Panelist 2 | pH 8.5 Panelist 2 | pH 5.5 Panelist 3 | pH 8.5 Panelist 3 |
| Control - water Example 10 | 17 | --- | --- | 0 | 0 | None | None | None | None | Slight- not offensive | Slight soapy- not offensive |
| | 18 | 0.2 | 39,900 | 0.4% | 5.5 | None | None | None | Trace Amine | Slight soapy not offensive | Moderate soapy- not offensive |
| Example 2 | 19 | 0.2 | 41,800 | 0.4% | 64 | None | Slight fishy | None | Slight amine | Moderate soapy not offensive | Considerable amine; offensive |

Odor panelists were asked to open each sample and assess the headspace for odor for 15 seconds. As shown by the results in Table 4, as expected, in bodywash formulated at a pH of 5.5-6.0, none of the panel members detected amine, fishy, or offensive odor, even in the bodywash Example 19 formulated with the untreated polymer of Example 2, which had a measured TMA level of 64 ppm.

In bodywash formulated at a pH of 8.0-8.5, all panel members detected amine, fishy, or offensive odor in bodywash Example 19, formulated with the untreated polymer of Example 2, which had a measured TMA level of 64 ppm. Only one of the panel members detected amine, fishy, or offensive odor in the bodywash Example 18 formulated with the polymer of the invention, Example 10, which had a measured TMA level of 6.5 ppm.

The combined results in Tables 3 and 4 demonstrate that personal care formulations at alkaline pH values containing the treated polymer , Example 10, have significantly reduced odor than personal care products containing untreated polymer, Example 2.

The samples of low molecular weight cationic guar polymer having reduced odor also show reduced odor in shampoo formulations, bodywash formulations, and other personal care products formulated at alkaline pH values, This same reduced odor performance for these reduced odor products are expected in household products, and pet care products that are formulated at alkaline pH values.

**Claims**

1. A process for preparing a reduced odor composition comprising (a) reacting at least one cationic polygalactomannan or cationic derivatized polygalactomannan with at least one reagent that reduces the Mw to less than 200,000 wherein the reaction mass includes water soluble color bodies and water insoluble material, (b) removing water insoluble material, and (c) removing odorous components, including trimethylamine (TMA) and other amines and low molecular weight components, in an aqueous phase by nitrogen sparging, distillation, ion exchange or membrane diafiltration to produce the reduced odor composition which comprises at least one cationic polygalactomannan or a derivative thereof having:

    (i) a weight average molecular weight (Mw) of 5,000 - 200,000,
    (ii) a light transmittance in a 10% aqueous solution of greater than 80% at a light wavelength of 600 nm,
    (iii) a protein content of less than 1.0% by weight of polysaccharide, and
    (iv) a trimethylamine content of less than 25 ppm in a 10% aqueous solution of the polymer.

2. A process according to Claim 1, wherein the reduced odor composition has an aldehyde functionality content of at least 0.01 meq/gram.

3. A process according to Claim 1, wherein the reduced odor composition has a boron content of less than 100 ppm per gram of polygalactomannan.

4. A process according to Claim 1, wherein the reduced odor composition has a cationic degree of substitution (DS) of 0.01 - 3.0.

5. A process according to Claim 4, wherein the reduced odor composition has a cationic DS of at least 0.1.

6. A process according to Claim 5, wherein the reduced odor composition has a cationic DS of at least 0.2.

7. A process according to Claim 4, wherein the reduced odor composition has a cationic DS of 2.0 or less.

8. A process according to Claim 7, wherein the reduced odor composition has a cationic DS of 1.0 or less.

9. A process according to Claim 1, wherein the derivative moiety on the cationic derivatized polygatactomannan is selected from the group consisting of alkyl, hydroxyalkyl, alkylhydroxyalkyl and carboxymethyl, wherein the alkyl has a carbon chain containing from 1 to 22 carbons and the hydroxyalkyl is hydroxyethyl, hydroxypropyl or hydroxybutyl.

10. A process according to Claim 1, wherein the poygalaaomannan is guar, locust bean, honey locus or flame tree.

11. A process according to Claim 1, wherein the cationic moiety is a quaternary ammonium compound.

12. A process according to Claim 11, wherein the quaternary ammonium compound is selected from the group consisting of 3-chloro-2-hydroxypropyltrimethylammonium chloride, 2,3-epoxy-propyltrimethylammonium chloride, 3-chloro-2-hydroxypropyltrimethylammonium bromide, 2,3-epoxy-propyltrimethylammonium bromide; glycidyltrimethylammonium chloride, glycidyltriethylammonium chloride, gylcidyltripropylammonium chloride, glycidylethyidimethylammonium chloride, glycidyldiethylmethylommonium chloride, and their corresponding bromides and iodides;3-chloro-2-hydroxypropyltrimethylammonium chloride, 3-chloro-2-hydroxypropyltriethylammonium chloride, 3-chloro-2-hydroxypropyltriropylammonmm chloride. 3-chloro-2-hydroxypropylethyldimethylammonium chloride, and their corresponding bromides and iodides; and halides of imidazoline ring containing compounds.

13. A process according to Claim 1, wherein the light transmittance of the reduced odor compositions is greater than 90%.

14. A process according to Claim 1, wherein the light transmittance of the reduced odor composition is greater than 95%.

15. A process according to Claim 1, wherein the protein content of the reduced odor composition is less than 0.5% by weight of polysaccharide.

16. A process according to Claim 1, wherein the Mw of the reduced odor composition is at least 20,000.

17. A process according to Claim 16, wherein the Mw of the reduced odor composition is at least 35,000.

18. A process according to Claim 17, wherein the Mw of the reduced odor composition is at least 50,000.

19. A process according to Claim 1, wherein the Mw of the reduced odor composition is 100,000 or less,

20. A process according to Claim 19, wherein the Mw of the reduced odor composition is 70,000 or less.

21. A process according to Claim 1, wherein the reduced odor compositions further comprises a colorant, a preservative, an antioxidant, an alpha or beta hydroxy acid, an activity erhancer, an emulsifier, a functional polymer, a viscosifying agent, an alcohol, a fat or fatty compound, an antimicrobial compound, a zinc pyrithione, a silicone material, a hydrocarbon polymer, an emollient, an oil, a surfactant, a suspending agent or a mixture of any thereof.

22. A process according to Claim 21, wherein the functional polymer is an anionic, hydrophabically-modified, and amphoteric acrylic acid copolymer, a vinylpyrrolidone homopolymer or copolymer, a cationic vinylpyrrofidone copolymer, a nonionic, cationic, anionic, or amphoteric, cellulosic polymer, an acrylamide homopolymer, a cationic, anionic, amphoteric or hydrophohically-modified acrylamide copolymer, a polyethylene glycol polymer or copolymer, a hydrophobically-modified polyether, a hydrophobically-modified polyetheracetal, a hydrophobically-modified polyetherurethane, an associative polymer, a hydrophobically-modified cellulosic polymer, a polyethyleneoxide-propylene oxide copolymer, a nonionic, anionic, hydrophobically-modified, amphoteric or cationic polysaccharide, chitosan, or a mixture of any thereof.

23. A process according to Claim 22, wherein the nonionic, cationic, anionic or amphoteric cellulosic polymer is hydroxyethylcellulose, hydroxypropylcellulose, hydroxypropylmethylcellulose, carboxymethycellulose, hydrophoblcally-modified carboxymethylcllulose, cationic hydroxyethylcellulose, cationic hydrophobically-modified hydroxyethyl cellulose, hydrophobically modified hydroxyethylcellulose, hydrophobically-modified hydroxypropylcelluslose, cationic hydrophobically-modified hydroxypropyl cellulose, cationic carboxymethylhydroxyethylcellulose, or cationic hydroxypropylcellulose.

24. A process according to Claim 22, wherein the nonionic, anlonic, hydrophobically modified, amphoteric or cationic polysaccharide is carboxymethyl guar, an alginate, hydroxypropyl guar, hydrophobically-modified guar, carboxymethyl guar hydroxypropyltrimethylammonium chloride, guar hydroxypropyltrimethylammonium chloride or hydroxypropyl guar hydroxypropyltrimethylammonium chloride.

25. A process according to Claim 21, wherein the visoasifying agent NaCl, $NH_4Cl$, KCl, $Na_2SO_4$, a fatty alcohol, a fatty acid ester, a fatty acid amide, a fatty alcohol polyethyleneglycol ether, a sorbitol polyethyleneglycol ether, cocamidopropyl betaine, a clay, a silica, a cellulosic polymer, xanthan or a mixture of any thereof.

26. A process according to Claim 21, wherein the silicone material is a cyclosiloxane, a linear siloxane, a siloxane structure with polyol, amino or other functional groups in the siloxane structure or a mixture of any thereof.

27. A process according to Claim 26, wherein the other functional groups are polyethyleneoxy and/or polypropyleneoxy groups optionally containing $C_6$-$C_{24}$ alkyl groups, substituted or unsubstituted amine groups, thiol groups, alkoxylated groups, hydroxyl groups or acylaxyalkyl groups.

28. A process according to Claim 21, wherein the silicone material is a polyalkylsiloxane, a polyarylsiloxane, a polyalkylarylsiloxane or a mixture of any thereof.

29. A process according to Claim 28, wherein the polyalkylsiloxane is a polydimethylsiloxane, a polydimethylsiloxane hydroxylated at the end of the chain or a mixture of any thereof.

30. A process according to Claim 21, wherein the surfactant is anionic, amphoteric or nonionic

31. A process according to Claim 9, wherein the cationic polygalactomannan or cationic derivatized palygatactornannan is treated with the reagent in an aqueous medium to produce an aqueous dispersion composed of an aqueous phase and a solid phase of the treated polygalactomannan, the water insoluble solid materials are removed from the dispersion eliminating the solid phase, and odorous components, including trimethylamine (TMA) and other amines and low molecular weight components are removed from the aqueous phase to produce a clarified solution of the reduce odor composition.

32. A process according to Claim 31, wherein the reagent is an oxidizing reagent selected from a peroxide, a persulfate, a permanganate, a perchlorate, a hypochlorite, oxygen, and a biochemical oxidant.

33. A process according to Claim 32, wherein the peroxide is hydrogen peroxide.

34. A process according to Claim 32, wherein the biochemical oxidizing reagent is an oxygenase.

35. A process according to Claim 34, wherein the oxygenase is galactose oxidase.

36. A proces according to Claim 31, wherein the reagent further comprises a hydrolytic reagent.

37. A process according to Claim 36, wherein said hydrolytic reagent is a hydrolytic enzyme,

38. A process according to Claim 37, wherein said hydrolytic enzyme is a hemicellulose.

39. A process according to Claim 38, wherein the hemicellulase is mannanase.

40. A process according to Claim 36, wherein said hydrolytic reagent is an organic or mineral acid.

41. A process according to Claim 31, further comprising removing the water soluble color bodies.

42. A process according to Claim 41, wherein the water soluble color bodies are removed by addition of sodium metabisulfite, sodium bisulfite, sodium hypochlorite or sodium chlorite.

43. A process according to Claim 41, wherein the water soluble color bodies are removed by addition of activated carbon, fallowed by a separation step.

44. A process according to Claim 41, wherein the water soluble color bodies are removed by addition of molecular sieves, followed by a separation step.

45. A process according to Claim 31, further comprising recovering the derivatized polygalactomannan in dry form from the aqueous solution.

46. A process according to Claim 1, wherein the cationic polygalatomannan or cationic derivatized polygalactomannan is in the form of powder, flour or splits.

47. A process according to Claim 1, wherein the membrane diafiltration uses nanofiltration membranes selected from the group consisting of hollow fiber, spiral wound, and plate and frame.

48. A process according to Claim 1, wherein the ion exchange process uses a polystyrene-based ion exchange resin.

49. A process according to Claim 1, wherein the nitrogen sparging is performed at atmospheric pressure or with the aid of vacuum.

50. A process according to Claim 1, wherein the distillation process includes general distillation or extractive distillation, using water as the extractive solvent

51. A process for preparing a composition, the process comprising the process according to claim 1 and further comprising the step of incorporating the reduced odor composition into a functional system selected from the group consisting of personal care product, household care product and pet care product.

52. A process according to Claim 51, wherein the reduced odor composition is incorporated into the functional system in an amount of less than 10 wt%.

53. A process according to Claim 52, wherein the reduced odor composition is incorporated into the functional system in an amount of less than 5 wt%.

54. A process according to Claim 53, wherein the reduced odor composition is incorporated into the functional system in an amount of less than 1 wt%.

55. A process according to Claim 51, wherein the functional system has an acidic pH.

56. A process according to Claim 51, wherein the functional system has a neutral pH.

57. A process according to Claim 51, wherein the functional system has an alkaline pH.

58. A process according to Claim 51, wherein the household product includes an insect repellent agent, a pet deodorizer agent, a pet shampoo active, an industrial grade bar and liquid soap active, a dishwashing soap actives, an all purpose cleaner, a disinfecting agent rug and upholstery cleaning active, laundry softener active, sundry detergent active, toilet bowl cleaning agent fabric sizing agent, dust collection agent, anfiredeposition agent, textile cleaning agent, and lubricating agent.

59. A process according to Claim 51, wherein the functional system is a household care or pet care product and which comprises at least one additional ingredient selected from the group consisting of colorant, preservative, antioxidant, bleaching agent, activity enhance, emulsifier, functional polymer, viscosifying agent, alcohol, fat or fatty compound, oil, surfactant, fragrance, suspending agent, silicone material, and mixtures thereof.

60. A process according to Claim 51, wherein the functional system is a personal care product including an active personal care ingredient selected from the group consisting of perfumes, skin coolants, emolfients, deodorants, antiperspirants actives, moisturizing agents, cleansing agents, sunscreen actives, hair treatment agents, oral care agents, denture adhesive agents, shaving actives, beauty aids, and nail care active.

61. A process according to Claim 51, wherein the personal care product is selected from the group consisting of hair care, skin care, sun care, nail care, and oral care.

62. A process according to Claim 61, wherein the personal care product is a hair care product comprising a conditioning agent selected from the group consisting of silicone materials, hydrocarbon oils, panthenol and derivatives thereof, pantothenic acid and derivatives thereof/and mixtures thereof.

63. A process according to Claim 61, wherein the personal care product is a skin care product comprising a conditioning agent selected from the group of consisting of silicone materials, hydrocarbon oils, panthenol and derivatives thereof, pantothenic acid and derivatives thereof, and mixtures thereof.

64. A process according to Claim 61, wherein the personal care product is a hair care product or skin care product comprising up to 5% by weight the reduced odor composition and has a light transmittance value greater than 95%.

65. A process according to Claim 51, wherein the functional system is a personal care product which comprises at least one additional ingredient selected from the group consisting of colorant, preservative, antioxidant, alpha or beta hydroxy acid, activity enhancer, emulsifier, functional polymer, viscosifying agent, alcohol, fat or fatty compound, antimicrobial compound, zinc pyrithione, silicone material, hydrocarbon polymer, emollient, oil, surfactant, flavour, fragrance, medicaments, rejunvenating agents, suspending agents, and mixture thereof.

**66.** A process according to Claim 51, wherein the functional system is an oil-in-water or water-in-oil emulsion.

**67.** A process according to Claim 1, wherein the reduced odor composition comprises water in an amount of 50 - 95% by weight of the total composition.

**68.** A process according to Claim 67, wherein the reduced odor composition further comprises at least one additional ingredient selected from the group consisting of stabilising biocides, fragrances, flavors, colorants, and mixtures thereof.

**Patentansprüche**

**1.** Verfahren zur Herstellung einer Zusammensetzung mit verringertem Geruch, umfassend die folgenden Schritte: (a) Umsetzen von mindestens einem kationischen Polygalactomannan oder einem kationischen derivatisierten Polygalactomannan mit mindestens einem Reagenz, das das MG auf weniger als 200.000 verringert, wobei der Reaktionsansatz wasserlösliche Farbkörper und wasserunlösliches Material beinhaltet, (b) Entfernen von wasserunlöslichem Material, und (c) Entfernen von Geruchskomponenten einschließlich Trimethylamin (TMA) und anderen Aminen und niedermolekularen Verbindungen, in einer wässrigen Phase durch Sparging mit Stickstoff, Destillation, Ionenaustausch oder Membrandiafiltration, wodurch man die Zusammensetzung mit verringertem Geruch erhält, die mindestens ein kationisches Polygalactomannan oder ein Derivat davon mit:

(i) einem gewichtsmittleren Molekulargewicht (MG) von 5.000-200.000,
(ii) einem Lichtdurchlassgrad in einer 10%igen wässrigen Lösung von mehr als 80% bei einer Lichtwellenlänge von 600 nm,
(iii) einem Proteingehalt von weniger als 1,0 Gew.-% Polysaccharid und
(iv) einem Trimethylamingehalt von weniger als 25 ppm in einer 10%igen wässrigen Lösung des Polymers umfasst.

**2.** Verfahren nach Anspruch 1, wobei die Zusammensetzung mit verringertem Geruch einen Aldehydfunktionsgehalt von mindestens 0,01 meq/Gramm aufweist.

**3.** Verfahren nach Anspruch 1, wobei die Zusammensetzung mit verringertem Geruch einen Borgehalt von weniger als 100 ppm pro Gramm Polygalactomannan aufweist.

**4.** Verfahren nach Anspruch 1, wobei die Zusammensetzung mit verringertem Geruch einen kationischen Substitutionsgrad (SG) von 0,01-3,0 aufweist.

**5.** Verfahren nach Anspruch 4, wobei die Zusammensetzung mit verringertem Geruch einen kationischen SG von mindestens 0,1 aufweist.

**6.** Verfahren nach Anspruch 5, wobei die Zusammensetzung mit verringertem Geruch einen kationischen SG von mindestens 0,2 aufweist.

**7.** Verfahren nach Anspruch 4, wobei die Zusammensetzung mit verringertem Geruch einen kationischen SG von 2,0 oder weniger aufweist.

**8.** Verfahren nach Anspruch 7, wobei die Zusammensetzung mit verringertem Geruch einen kationischen SG von 1,0 oder weniger aufweist.

**9.** Verfahren nach Anspruch 1, wobei der Derivatrest des kationischen derivatisierten Polygalactomannans aus der Gruppe Alkyl, Hydroxyalkyl, Alkylhydroxyalkyl und Carboxymethyl ausgewählt ist, wobei das Alkyl eine Kohlenstoffkette mit 1 bis 22 Kohlenstoffen aufweist und es sich bei dem Hydroxyalkyl um flydroxyethyl, Hydroxypropyl oder Hydroxybutyl handelt.

**10.** Verfahren nach Anspruch 1, wobei es sich bei dem Polygalactomannan um Guar, Johannisbrot, Lederhülsenbaum oder Flammenbaum handelt.

**11.** Verfahren nach Anspruch 1, wobei es sich bei dem kationischen Rest um eine quartäre Ammoniumverbindung handelt.

12. Verfahren nach Anspruch 11, wobei die quartäre Ammoniumverbindung aus der Gruppe 3-Chlor-2-hydroxypropyltrimethylammoniumchlorid, 2,3-Epoxypropyltrimethylammoniumchlorid, 3-Chlor-2-hydroxypropyltrimethylammoniumbromid, 2,3-Epoxypropyltrimethylammoniumbromid; Glycidyltrimethylammoniumchlorid, Glycidyltriethylammoniumchlorid, Glycidyltripropylammoniumchlorid, Glycidylethyldimethylammoniumchlorid, Glycidyldiethylmethylammoniumchlorid und ihren entsprechenden Bromiden und Iodiden; 3-Chlor-2-hydroxypropyltrimethylammoniumchlorid, 3-Chlor-2-hydroxypropyltriethylammoniumchlorid, 3-Chlor-2-hydroxypropyltripropylammoniumchlorid, 3-Chlor-2-hydroxypropylethyldimethylammoniumchlorid und ihren entsprechenden Bromiden und Iodiden; und Halogeniden von imidazolringhaltigen Verbindungen ausgewählt ist.

13. Verfahren nach Anspruch 1, wobei der Lichtdurchlassgrad der Zusammensetzung mit verringertem Geruch über 90% beträgt.

14. Verfahren nach Anspruch 1, wobei der Lichtdurchlassgrad der Zusammensetzung mit verringertem Geruch über 95% beträgt.

15. Verfahren nach Anspruch 1, wobei der Proleingehalt der Zusammensetzung mit verringertem Geruch weniger als 0,5 Gew.-% des Polysaccharids beträgt.

16. Verfahren nach Anspruch 1, wobei das MG der Zusammensetzung mit verringertem Geruch mindestens 20.000 beträgt.

17. Verfahren nach Anspruch 16, wobei das MG der Zusammensetzung mit verringertem Geruch mindestens 35.000 beträgt.

18. Verfahren nach Anspruch 17, wobei das MG der Zusammensetzung mit verringertem Geruch mindestens 50.000 beträgt.

19. Verfahren nach Anspruch 1, wobei das MG der Zusammensetzung mit verringertem Geruch 100.000 oder weniger beträgt.

20. Verfahren nach Anspruch 19, wobei das MG der Zusammensetzung mit verringertem Geruch 70.000 oder weniger beträgt.

21. Verfahren nach Anspruch 1, wobei die Zusammensetzung mit verringertem Geruch weiterhin einen Farbstoff, ein Konservierungsmittel, ein Antioxidationsmittel, eine alpha- oder beta-Hydroxysäure, einen Wirkungsverstärker, einen Emulgator, ein funktionelles Polymer, ein Verdickungsmittel, einen Alkohol, ein Fett oder eine Fettverbindung, eine antimikrobielle Verbindung, ein Zinkpyrithion, ein Siliconmaterial, ein Kohlenwasserstoffpolymer, ein Erweichungsmittel, ein Öl, ein Tensid, ein Suspendiermittel oder eine Mischung von beliebigen dieser Stoffe umfasst.

22. Verfahren nach Anspruch 21, wobei es sich bei dem funktionellen Polymer um ein anionisches, hydrophob modifiziertes und amphoteres Acrylsäurecopolymer, ein Vinylpyrrolidonhomopolymer oder -copolymer, ein kationisches Vinylpyrrolidoncopolymer, ein nichtionisches, kationisches, anionisches oder amphoteres Zellulosepolymer, ein Acrylamidhomopolymer, ein kationisches, anionisches, amphoteres oder hydrophob modifiziertes Acrylamidcopolymer, ein Polyethylenglykolpolymer oder -copolymer, einen hydrophob modifizierten Polyether, einen hydrophob modifizierten Polyetheracetal, ein hydrophob modifiziertes Polyetherurethan, ein Assoziativpolymer, ein hydrophob modifiziertes Cellulosepolymer, ein Polyethylenoxid-Propylenoxid-Copolymer, ein nichtionisches, anionisches, hydrophob modifiziertes, amphoteres oder kationisches Polysaccharid, Chitosan oder eine Mischung von beliebigen dieser Substanzen handelt.

23. Verfahren nach Anspruch 22, wobei es sich bei dem nichtionischen, kationischen, anionischen oder amphoteren Cellulosepolymer um Hydroxyethylcellulose, Hydroxypropylcellulose, Hydroxypropylmethylcellulose, Carboxymethylcellulose, hydrophob modifizierte Carboxymethylcellulose, kationische Hydroxyethylcellulose, kationische hydrophob modifizierte Hydroxyethylcellulose, hydrophob modifizierte Hydroxyethylcellulose, hydrophob modifizierte Hydroxypropylcellulose, kationische hydrophob modifizierte Hydroxypropylcellulose, kationische Carboxymethylhydroxyethylcellulose oder kationische Hydroxypropylcellulose handelt.

**24.** Verfahren nach Anspruch 22, wobei es sich bei dem nichtionischen, anionischen, hydrophob modifizierten, amphoteren oder kationischen Polysaccharid um Carboxymethylguar, ein Alginat, Hydroxypropylguar, hydrophob modifiziertes Guar, Carboxymethylguar-Hydroxypropyltrimethylammoniumchlorid, Guar-HydroxypropyltrimeLhylamnoniumchlorid oder Hydroxypropylguar-Hydroxypropyltrimethylammoniumchlorid handelt.

**25.** Verfahren nach Anspruch 21, wobei es sich bei dem Verdickungsmittel um NaCl, NH$_4$Cl, KCl, Na$_2$SO$_4$, einen Fettalkohol, einen Fettsäureester, ein Fettsäureamid, einen Fettalkoholpolyethylenglykolether, einen Sorbitpolyethylenglykolether, Cocamidopropylbetain, einen Ton, ein Siliziumdioxid, ein Cellulosepolymer, Xanthan oder eine Mischung von beliebigen dieser Substanzen handelt.

**26.** Verfahren nach Anspruch 21, wobei es sich bei dem Siliconmaterial um ein Cyclosiloxan, ein lineares Siloxan, eine Siloxanstruktur mit Polyolgruppen, Aminogruppen oder anderen funktionellen Gruppen in der Siloxanstruktur oder um eine Mischung von beliebigen dieser Substanzen handelt.

**27.** Verfahren nach Anspruch 26, wobei es sich bei den anderen funktionellen Gruppen um Polyethylenoxy-und/oder Polypropylenoxygruppen, gegebenenfalls enthaltend C$_6$-C$_{24}$-Alkylgruppen, substituierte oder unsubstituierte Amingruppen, Thiolgruppen, alkoxylierte Gruppen, Hydroxylgruppen oder Acyloxyalkylgruppen handelt.

**28.** Verfahren nach Anspruch 21, wobei es sich bei dem Siliconmaterial um ein Polyalkylsiloxan, ein Polyarylsiloxan, ein Polyalkylarylsiloxan oder eine Mischung von beliebigen dieser Substanzen handelt.

**29.** Verfahren nach Anspruch 28, wobei es sich bei dem Polyalkylsiloxan um ein Polydimethylsiloxan, ein am Ende der Kette hydroxyliertes Polydimethylsiloxan oder eine Mischung von beliebigen dieser Substanzen handelt.

**30.** Verfahren nach Anspruch 21, wobei das Tensid anionisch, amphoter oder nichtionisch ist.

**31.** Verfahren nach Anspruch 1, wobei das kationische Polygalactomannan oder kationisch derivatisierte Polygalactomannan mit dem Reagenz in einem wässrigen Medium behandelt wird, wodurch man zu einer wässrigen Dispersion gelangt, die aus einer wässrigen Phase und einer festen Phase des behandelten Polygalactomannans besteht, die wasserunlöslichen festen Substanzen von der Dispersion abgetrennt werden, wodurch man die feste Phase entfernt, und Geruchskomponenten, einschließlich Trimethylamin (TMA) und andere Amine und niedermolekulare Komponenten von der wässrigen Phase abgetrennt werden, wodurch man zu einer geklärten Lösung der Zusammensetzung mit verringertem Geruch gelangt.

**32.** Verfahren nach Anspruch 31, wobei es sich bei dem Reagenz um ein Oxidationsmittel, ausgewählt aus einem Peroxid, einem Persulfat, einem Permanganat, einem Perchlorat, einem Hypochlorit, Sauerstoff und einem biochemischen Oxidationsmittel handelt.

**33.** Verfahren nach Anspruch 32, wobei es sich bei dem Peroxid um Wasserstoffperoxid handelt.

**34.** Verfahren nach Anspruch 32, wobei es sich bei dem biochemischen Oxidationsreagenz um eine Oxygenase handelt.

**35.** Verfahren nach Anspruch 31, wobei es sich bei der Oxygenase um Galactoseoxidase handelt.

**36.** Verfahren nach Anspruch 31, wobei das Reagenz weiterhin ein hydrolytisches Reagenz umfasst.

**37.** Verfahren nach Anspruch 36, wobei es sich bei dem hydrolytischen Reagenz um ein hydrolytisches Enzym handelt.

**38.** Verfahren nach Anspruch 37, wobei es sich bei dem hydrolytischen Enzym um eine Hemicellulase handelt.

**39.** Verfahren nach Anspruch 38, wobei es sich bei der Hemicellulase um Mannanase handelt.

**40.** Verfahren nach Anspruch 36, wobei es sich bei dem hydrolytischen Reagenz um eine organische Säure oder eine Mineralsäure handelt.

**41.** Verfahren nach Anspruch 31, das weiterhin das Entfernen der wasserlöslichen Farbkörper umfasst.

**42.** Verfahren nach Anspruch 41, wobei die wasserlöslichen Farbkörper durch Versetzen mit Natriummetabisulfit, Natriumbisulfit, Natriumhypochlorit oder Natriumchlorit entfernt werden.

43. Verfahren nach Anspruch 41, wobei die wasserlöslichen Farbkörper durch Versetzen mit Aktivkohle und einem anschließenden Trennungsschritt entfernt werden.

44. Verfahren nach Anspruch 41, wobei die wasserlöslichen Farbkörper durch Versetzen mit Molekularsieb und einem anschließenden Trennungsschritt entfernt werden.

45. Verfahren nach Anspruch 31, das weiterhin das Gewinnen des derivatisierten Polygalactomannans in trockener Form aus der wässrigen Lösung umfasst.

46. Verfahren nach Anspruch 1, wobei das kationische Polygalactomannan oder kationische derivatisierte Polygalactomannan in Form von Pulver, Mehl oder Splits vorliegt.

47. Verfahren nach Anspruch 1, wobei bei der Membrandiafiltration Nanofiltrationsmembranen aus der Gruppe Hohlfasermembran, Spiralwickelmembran und Platte und Rahmen eingesetzt werden.

48. Verfahren nach Anspruch 1, wobei bei dem Ionenaustauschverfahren ein Ionenaustauschharz auf Polystyrolbasis eingesetzt wird.

49. Verfahren nach Anspruch 1, wobei das Sparging mit Stickstoff bei Atmosphärendruck oder mit Hilfe eines Vakuums erfolgt.

50. Verfahren nach Anspruch 1, wobei das Destillationsverfahren die Destillation im Allgemeinen oder die Extraktivdestillation mit Wasser als Extraktionslösungsmittel beinhaltet.

51. Verfahren zur Herstellung einer Zusammensetzung, wobei das Verfahren das Verfahren nach Anspruch 1 umfasst und weiterhin den Schritt des Einarbeitens der Zusammensetzung mit verringerlem Geruch in ein funktionelles System aus der Gruppe Körperpflegeprodukt, Haushaltspflegeprodukt und Haustierpflegeprodukt umfasst.

52. Verfahren nach Anspruch 51, wobei die Zusammensetzung mit verringertem Geruch in das funktionelle System in einer Menge von weniger als 10 Gew.-% eingearbeitet wird.

53. Verfahren nach Anspruch 52, wobei die Zusammensetzung mit verringertem Geruch in das funktionelle System in einer Menge von weniger als 5 Gew.-% eingearbeitet wird.

54. Verfahren nach Anspruch 53, wobei die Zusammensetzung mit verringertem Geruch in das funktionelle System in einer Menge von weniger als 1 Gew.-% eingearbeitet wird.

55. Verfahren nach Anspruch 51, wobei das funktionelle System einen sauren pH-Wert aufweist.

56. Verfahren nach Anspruch 51, wobei das funktionelle System einen neutralen pH-Wert aufweist.

57. Verfahren nach Anspruch 51, wobei das funktionelle System einen alkalischen pH-Wert aufweist.

58. Verfahren nach Anspruch 51, wobei das Haushaltsprodukt ein Insektenabwehrmittel, ein Mittel zum Entfernen von Gerüchen bei Haustieren, einen Haustiershampoowirkstoff, einen Wirkstoff für Seifenriegel und Flüssigseile in technischer Qualität, einen Geschirrspülmittelwirkstoff, einen Allzweckreiniger, ein Desinfektionsmittel, einen Reinigungswirkstoff für Teppiche und Polstermöbel, einen Weichmacherwirkstoff für Wäsche, einen Reinigungswirkstoff für Wäsche, ein WC-Reinigungsmittel, eine Appretur für Stoffe, ein Staubsammelmittel, ein Mittel gegen die Wiederablagerung, ein Textilreinigungsmittel und ein Gleitmittel beinhaltet.

59. Verfahren nach Anspruch 51, wobei es sich bei dem funktionellen System um ein Haushaltspflegemittel oder ein Haustierpflegemittel handelt, das mindestens einen zusätzlichen Wirkstoff, ausgewählt aus der Gruppe Farbstoff, Konservierungsmittel, Antioxidationsmittel, Bleichmittel, Wirkungsverstärker, Emulgator, funktionelles Polymer, Verdickungsmittel, Alkohol, Fett oder Fettverbindung, Öl, Tensid, Duftstoff, Suspendiermittel, Siliconmaterial und Mischungen davon umfasst.

**60.** Verfahren nach Anspruch 51, wobei es sich bei dem funktionellen System um ein Körperpflegemittel handelt, das einen Körperpflegewirkstoff aus der Gruppe Parfums, hautkühlende Mittel, zartmachende Mittel, Deodorantien, Antiperspirant-Wirkstoffe, feuchtigkeitsspendende Mittel, reinigende Mittel, Sonnenschutzwirkstoffe, Haarbehandlungsmittel, Mundpflegemittel, Zahnprotesenhaftmittel, Rasierwirkstoffe, Schönheitsmittel und Nagelpflegewirkstoffe beinhaltet.

**61.** Verfahren nach Anspruch 51, wobei das Körperpflegemittel aus der Gruppe Haarpflege, Hautpflege, Sonnenpflege, Nagelpflege und Mundpflege stammt.

**62.** Verfahren nach Anspruch 61, wobei es sich bei dem Körperpflegemittel um ein Haarpflegemittel, umfassend ein Konditioniermittel aus der Gruppe Siliconmaterialien, Kohlenwasserstofföle, Panthenol und ihre Derivate, Pantothensäure und ihre Derivate und Mischungen dieser Substanzen handelt.

**63.** Verfahren nach Anspruch 61, wobei es sich bei dem Körperpflegemittel um ein Hautpflegemittel, umfassend ein Konditioniermittel aus der Gruppe Siliconmaterialien, Kohlenwasserstofföle, Panthenol und ihre Derivate, Pantothensäure und ihre Derivate und Mischungen dieser Substanzen handelt.

**64.** Verfahren nach Anspruch 61, wobei es sich bei dem Körperpfegemittel um ein Haarpflegemittel handelt, das bis zu 5 Gew. -% der Zusammensetzung mit verringertem Geruch umfasst und das einen Lichtdurchlasswert von mehr als 95% aufweist.

**65.** Verfahren nach Anspruch 51, wobei es sich bei dem funktionellen System um ein Körperpflegemittel handelt, das mindestens einen zusätzlichen Wirkstoff, ausgewählt aus der Gruppe Farbstoff, Konservierungsmittel, Antioxidationsmittel, alpha- oder beta-Hydroxysäure, Wirkungsverstärker, Emulgator, funktionelles Polymer, Verdickungsmittel, Alkohol, Fett oder Fettverbindung, antimikrobielle Verbindung, Zinkpyrithion, Siliconmaterial, Kohlenwasserstoffpolymer, Emollientium, Öl, Tensid, Aroma, Duftstoff, Medikamente, verjüngende Mittel, Suspendiermittel und Mischung davon umfasst.

**66.** Verfahren nach Anspruch 51, wobei es sich bei dem funktionellen System um eine Öl-in-Wasser- oder Wasserin-Öl-Emulsion handelt.

**67.** Verfahren nach Anspruch 1, wobei die Zusammensetzung mit verringertem Geruch Wasser in einer Menge von 50-95 Gew.-% der Gesamtzusammensetzung umfasst.

**68.** Verfahren nach Anspruch 67, wobei die Zusammensetzung mit verringertem Geruch weiterhin mindestens einen zusätzlichen Wirkstoff aus der Gruppe stabilisierende Biozide, Duftstoffe, Aromen, Farbstoffe und Mischungen davon umfasst.

**Revendications**

**1.** Procédé de préparation d'une composition à odeur réduite, comprenant (a) la réaction d'au moins un polygalactomannane cationique ou un polygalactomannane dérivatisé cationique avec au moins un réactif qui réduit le PM jusqu'à moins de 200 000, où la masse réactionnelle comporte des corps colorés hydrosolubles et des matériaux insolubles dans l'eau, (b) l'élimination des matériaux insolubles dans l'eau, et (c) l'élimination des composants odorants, y compris la triméthylamine (TMA) et autres amines et composants de bas poids moléculaire, dans une phase aqueuse par barbotage d'azote, distillation, échange d'ions ou diafiltration sur membrane, pour produire la composition à odeur réduite comprenant au moins un polygalactomannane cationique ou un dérivé de celui-ci ayant :

      (i) un poids moléculaire moyen en poids (PM) de 5000-200 000,
      (ii) une transmittance de la lumière dans une solution aqueuse à 10% supérieure à 80% à une longueur d'onde de lumière de 600 nm,
      (iii) une teneur en protéines inférieure à 1,0% en poids de polysaccharide, et
      (iv) une teneur en triméthylamine inférieure à 25 ppm dans une solution aqueuse à 10% de polymère.

**2.** Procédé selon la revendication 1, dans lequel la composition à odeur réduite possède une teneur en fonctionnalités aldéhyde d'au moins 0,01 méq./gramme.

3. Procédé selon la revendication 1, dans lequel la composition à odeur réduite possède une teneur en bore inférieure à 100 ppm par gramme de polygalactomannane.

4. Procédé selon la revendication 1, dans lequel la composition à odeur réduite possède un taux de substitution cationique de (DS) de 0,01-3,0.

5. Procédé selon la revendication 4, dans lequel la composition à odeur réduite possède un DS cationique d'au moins 0,1.

6. Procédé selon la revendication 5, dans lequel la composition à odeur réduite possède un DS cationique d'au moins 0,2.

7. Procédé selon la revendication 4, dans lequel la composition à odeur réduite possède un DS cationique de 2,0 ou moins.

8. Procédé selon la revendication 7, dans lequel la composition à odeur réduite possède un DS cationique de 1,0 ou moins.

9. Procédé selon la revendication 1, dans lequel le motif de dérivé sur le polygalactomannane dérivatisé cationique est choisi dans le groupe constitué par alkyle, hydroxyalkyle, alkylhydroxyalkyle, et carboxyméthyle, où alkyle possède une chaîne carbonée contenant de 1 à 22 carbones et hydroxyalkyle est hydroxyéthyle, hydroxypropyle ou hydroxybutyle.

10. Procédé selon la revendication 1, dans lequel le polygalactomannane est issu de guar, de caroube, de robinier, ou de flamboyant.

11. Procédé selon la revendication 1, dans lequel le motif cationique est un composé d'ammonium quaternaire.

12. Procédé selon la revendication 11, dans lequel le composé d'ammonium quaternaire est choisi dans le groupe constitué par le chlorure de 3-chloro-2-hydroxypropyltriméthylammonium, le chlorure de 2,3-époxypropyltrimethy-lammonium, le bromure de 3-chloro-2-hydroxypropyltriméthylammonium, le bromure de 2,3-époxypropyltriméthylammonium, le chlorure de glycidyltriméthylammonium, le chlorure de glycidyltriéthylammonium, le chlorure de gylcidyltripropylammonium, le chlorure de glycidyléthyldiméthylammonium, le chlorure de glycidyldiéthylméthylammonium, et leurs bromures et iodures correspondants ; le chlorure de 3-chloro-2-hydroxypropyltriméthylammonium, le chlorure de 3-chloro-2-hydroxypropyltriéthylammonium, le chlorure de 3-chloro-2-hydroxypropyltripropylammonium, le chlorure de 3-chloro-2-hydroxypropyléthyldiméthylammonium, et leurs bromures et iodures correspondants ; et les halogénures de composés à cycle imidazoline.

13. Procédé selon la revendication 1, dans lequel la transmittance de la lumière de la composition à odeur réduite est supérieure à 90%.

14. Procédé selon la revendication 1, dans lequel la transmittance de la lumière de la composition à odeur réduite est supérieure à 95%.

15. Procédé selon la revendication 1, dans lequel la teneur en protéines de la composition à odeur réduite est inférieure à 0,5% en poids de polysaccharide.

16. Procédé selon la revendication 1, dans lequel le PM de la composition à odeur réduite est d'au moins 20 000.

17. Procédé selon la revendication 16, dans lequel le PM de la composition à odeur réduite est d'au moins 35 000.

18. Procédé selon la revendication 17, dans lequel le PM de la composition à odeur réduite est d'au moins 50 000.

19. Procédé selon la revendication 1, dans lequel le PM de la composition à odeur réduite est de 100 000 ou moins.

20. Procédé selon la revendication 19, dans lequel le PM de la composition à odeur réduite est de 70 000 ou moins.

21. Procédé selon la revendication 1, dans lequel la composition à odeur réduite comprend en outre un colorant, un conservateur, un antioxydant, un acide alpha- ou bêta-hydroxylé, un améliorant d'activité, un émulsifiant, un polymère fonctionnel, un agent de viscosité, un alcool, une matière grasse ou un composé gras, un composé antimicrobien, de la pyrithione de zinc, un matériau siliconé, un polymère hydrocarboné, un émollient, une huile, un agent tensioactif, un agent de suspension ou un mélange quelconque de ceux-ci.

22. Procédé selon la revendication 21, dans lequel le polymère fonctionnel est un copolymère d'acide acrylique anionique, hydrophobiquement modifié, et amphotère ; un homopolymère ou copolymère de vinylpyrrolidone ; un copolymère de vinylpyrrolidone cationique ; un polymère cellulosique non ionique, cationique, anionique, ou amphotère ; un homopolymère d'acrylamide ; un copolymère d'acrylamide cationique, anionique, amphotère, ou hydrophobiquement modifié ; un polymère ou copolymère de polyéthylène glycol ; un polyéther hydrophobiquement modifié ; un polyétheracétal hydrophobiquement modifié ; un polyétheruréthane hydrophobiquement modifié ; un polymère associatif ; un polymère cellulosique hydrophobiquement modifié ; un copolymère d'oxyde de polyéthyléne-oxyde de propylène ; un pulysaccharide non ionique, anionique, hydrophobiquement modifié, amphotère, ou cationique ; le chitosane ; ou un mélange quelconque de ceux-ci.

23. Procédé selon la revendication 22, dans lequel le polymère cellulosique non ionique, cationique, anionique, ou amphotère est l'hydroxyéthylcellulose, l'hydroxypropylcellulose, l'hydroxypropyl méthylcellulose, la carboxyméthylcellulose, la carboxyméthylcellulose hydrophobiquement modifiée, l'hydroxyethylcellulose cationique, l'hydroxyéthyl cellulose cationique hydrophobiquement modifiée, l'hydroxyéthylcellulose hydrophobiquement modifiée, l'hydroxypropylcellulose hydrophobiquement modifiée, l'hydroxypropyl cellulose cationique hydrophobiquement modifiée, la carboxyméthyl hydroxyéthylcellulose cationique, ou l'hydroxypropylcellulose cationique.

24. Procédé selon la revendication 22, dans lequel le polysaccharide non ionique, anionique, hydrophobiquement modifié, amphotère, ou cationique est le carboxyméthyl guar, un alginate, l'hydroxypropyl guar, le guar hydrophobiquement modifié, le chlorure d'hydroxypropyltriméthylammonium carboxyméthyl guar, le chlorure d'hydroxypropyltriméthylammonium guar, ou le chlorure d'hydroxypropyltriméthylammonium hydroxypropyl guar.

25. Procédé selon la revendication 21 , dans lequel l'agent de viscosité est NaCl, $NH_4Cl$, KCl, $Na_2SO_4$, un alcool gras, un ester d'acides gras, un amide d'acides gras, un éther de polyéthylène glycol et d'alcool gras, un éther de polyéthylène glycol et de sorbitol, la cocamidopropyl bétaïne, une argile, une silice, un polymère cellulosique, un xanthane, ou un mélange quelconque de ceux-ci.

26. Procédé selon la revendication 21, dans lequel le matériau siliconé est un cyclosiloxane, un siloxane linéaire, une structure de siloxane avec du polyol, amino, d'autres groupements fonctionnels dans la structure de siloxane, ou un mélange quelconque de ceux-ci.

27. Procédé selon la revendication 26, dans lequel les autres groupements fonctionnels sont des groupements polyéthylèneoxy et/ou polypropyléneoxy contenant éventuellement des groupements $C_6$-$C_{24}$ alkyle, des groupements amine substitués ou non substitués, des groupements thiol, des groupements alcoxy, des groupements hydroxyle, ou des groupements acyloxyalkyle.

28. Procédé selon la revendication 21, dans lequel le matériau siliconé est un polyalkylsiloxane, un polyarylsiloxane, un polyalkylarylsiloxane, ou un mélange quelconque de ceux-ci.

29. Procédé selon la revendication 28, dans lequel le polyalkylsiloxane est un polydiméthylsiloxane, un polydiméthylsiloxane hydroxylé à l'extrémité de la chaîne, ou un mélange quelconque de ceux-ci.

30. Procédé selon la revendication 21, dans lequel l'agent tensioactif est anionique, amphotère, ou non ionique.

31. Procédé selon la revendication 1, dans lequel le polygalactomannane cationique ou le polygalactomannane cationique dérivatisé est traité par le réactif dans un milieu aqueux pour produire une dispersion aqueuse composée d'une phase aqueuse et d'une phase solide du polygalactomannane traité, les matériaux solides insolubles dans l'eau sont éliminés de la dispersion, éliminant la phase solide, et les composants odorants, y compris la triméthylamine (TMA) et autres amines et composants de bas poids moléculaire sont éliminés de la phase aqueuse pour produire une solution clarifiée de la composition à odeur réduite.

32. Procédé selon la revendication 31, dans lequel le réactif est un réactif oxydant choisi parmi un peroxyde, un persulfate, un permanganate, un perchlorate, un hypochlorite, l'oxygène, et un oxydant biochimique.

**33.** Procédé selon la revendication 32, dans lequel le peroxyde est le peroxyde d'hydrogène.

**34.** Procédé selon la revendication 32, dans lequel le réactif oxydant biochimique est une oxygénase.

**35.** Procédé selon la revendication 34, dans lequel l'oxygénase est la galactose oxydase.

**36.** Procédé selon la revendication 31, dans lequel le réactif comprend en outre un réactif hydrolytique.

**37.** Procédé selon la revendication 36, dans lequel ledit réactif hydrolytique est une enzyme hydrolytique.

**38.** Procédé selon la revendication 37, dans lequel ladite enzyme hydrolytique est une hémicellulase.

**39.** Procédé selon la revendication 38, dans lequel l'hémicellulase est la mannanase.

**40.** Procédé selon la revendication 36, dans lequel ledit réactif hydrolytique est un acide organique ou minéral.

**41.** Procédé selon la revendication 31, comprenant en outre l'élimination des corps colorés hydrosolubles.

**42.** Procédé selon la revendication 41, dans lequel les corps colorés hydrosolubles sont éliminés par addition de métabisulfite de sodium, de bisulfite de sodium, d'hypochlorite de sodium, ou de chlorite de sodium.

**43.** Procédé selon la revendication 41, dans lequel les corps colorés hydrosolubles sont éliminés par addition de charbon actif, suivie d'une étape de séparation.

**44.** Procédé selon la revendication 41, dans lequel les corps colorés hydrosolubles sont éliminés par addition de tamis moléculaires, suivie d'une étape de séparation.

**45.** Procédé selon la revendication 31, comprenant en outre la récupération du polygalactomannane dérivatisé sous forme sèche à partir de la solution aqueuse.

**46.** Procédé selon la revendication 1, dans lequel le polygalactomannane cationique ou le polygalactomannane dérivatisé cationique se trouve sous la forme de poudre, de farine ou de cupules.

**47.** Procédé selon la revendication 1, dans lequel la diafiltration sur membrane utilise des membranes de nanofiltration choisies dans le groupe constitué par les configurations en fibre creuse, en spirale, ou en plaque et cadre.

**48.** Procédé selon la revendication 1, dans lequel le procédé d'échange d'ions utilise une résine d'échange d'ions à base de polystyrène.

**49.** Procédé selon la revendication 1, dans lequel le barbotage d'azote est effectué à pression atmosphérique ou à l'aide de vide.

**50.** Procédé selon la revendication 1, dans lequel le procédé de distillation comporte une distillation générale ou une distillation extractive, en utilisant de l'eau comme solvant d'extraction.

**51.** Procédé de préparation d'une composition, le procédé comprenant le procédé selon la revendication 1 et comprenant en outre l'étape d'incorporation de la composition à odeur réduite dans un système fonctionnel choisi dans le groupe constitué par un produit de soin personnel, un produit ménager et un produit de soin pour animaux familiers.

**52.** Procédé selon la revendication 51, dans lequel la composition à odeur réduite est incorporée dans le système fonctionnel selon une quantité inférieure à 10% en poids.

**53.** Procédé selon la revendication 52, dans lequel la composition à odeur réduite est incorporée dans le système fonctionnel selon une quantité inférieure à 5% en poids.

**54.** Procédé selon la revendication 53, dans lequel la composition à odeur réduite est incorporée dans le système fonctionnel selon une quantité inférieure à 1% en poids.

**55.** Procédé selon la revendication 51, dans lequel le système fonctionnel possède un pH acide.

**56.** Procédé selon la revendication 51, dans lequel le système fonctionnel possède un pH neutre.

**57.** Procédé selon la revendication 51, dans lequel le système fonctionnel possède un pH alcalin.

**58.** Procédé selon la revendication 51, dans lequel le produit ménager comporte un agent répulsif d'insectes, un désodorisant pour animaux familiers, une substance active de shampoing pour animaux familiers, une substance active de savon de qualité industrielle et de savon liquide, une substance active de détergent pour lave-vaisselle, un agent nettoyant multi-usages, un agent désinfectant, une substance active de nettoyage de tapis et de tissus d'ameublement, une substance active d'assouplissant pour le linge, une substance active de détergent pour le linge, un agent de nettoyage de cuvette de toilettes, un produit d'encollage pour tissus, un agent dépoussiérant, un agent anti-redéposition, un agent de nettoyage de textiles, et un agent lubrifiant.

**59.** Procédé selon la revendication 51, dans lequel le système fonctionnel est un produit ménager ou de soin pour animaux familiers, et qui comprend au moins un ingrédient supplémentaire choisi dans le groupe constitué par les colorants, les conservateurs, les antioxydants, les agents de blanchiment, les améliorants d'activité, les émulsifiants, les polymères fonctionnels, les agents de viscosité, les alcools, les matières grasses ou les composés gras, les huiles, les agents tensioactifs, les parfums, les agents de suspension, les matériaux siliconés, ou un mélange quelconque de ceux-ci.

**60.** Procédé selon la revendication 51, dans lequel le système fonctionnel est un produit de soin personnel comportant un ingrédient actif de soin personnel choisi dans le groupe constitué par les parfums, les rafraîchissants de la peau, les émollients, les déodorants, les substances actives anti-transpirantes, les agents hydratants, les agents nettoyants, les ingrédients actifs d'écran solaire, les agents de traitement capillaire, les agents de soin de la bouche, les adhésifs pour prothèses dentaires, les produits de rasage, les produits de maquillage, et les produits de soin des ongles.

**61.** Procédé selon la revendication 51, dans lequel le produit de soin personnel est choisi dans le groupe constitué par le soin des cheveux, le soin de la peau, le soin solaire, le soin des ongles et le soin de la bouche.

**62.** Procédé selon la revendication 61, dans lequel le produit de soin personnel est un produit de soin des cheveux comprenant un agent de conditionnement choisi dans le groupe constitué par les matériaux siliconés, les huiles hydrocarbonées, le panthénol et ses dérivés, l'acide pantothénique et ses dérivés, et des mélanges de ceux-ci.

**63.** Procédé selon la revendication 61, dans lequel le produit de soin personnel est un produit de soin de la peau comprenant un agent de conditionnement choisi dans le groupe constitué par les matériaux siliconés, les huiles hydrocarbonées, le panthénol et ses dérivés, l'acide pantothénique et ses dérivés, et des mélanges de ceux-ci.

**64.** Procédé selon la revendication 61, dans lequel le produit de soin personnel est un produit de soin des cheveux ou un produit de soin de la peau comprenant jusqu'à 5% en poids de composition à odeur réduite et possède une valeur de transmittance de la lumière supérieure à 95%.

**65.** Procédé selon la revendication 51, dans lequel le système fonctionnel est un produit de soin personnel comprenant au moins un ingrédient supplémentaire choisi dans le groupe constitué par les colorants, les conservateurs, les antioxydants, les acides alpha- et bêta-hydroxylés, les améliorants d'activité, les émulsifiants, les polymères fonctionnels, les agents de viscosité, les alcools, les matières grasses ou les composés gras, les composés antimicrobiens, la pyrithione de zinc, les matériaux siliconés, les polymères hydrocarbonés, les émollients, les huiles, les agents tensioactifs, les parfums, les fragrances, les médicaments, les agents revitalisants, les agents de suspension, et des mélanges de ceux-ci.

**66.** Procédé selon la revendication 51, dans lequel le système fonctionnel est une émulsion huile-dans-eau ou eau-dans-huile.

**67.** Procédé selon la revendication 1, dans lequel la composition à odeur réduite comprend de l'eau selon une quantité de 50-95% en poids de la composition totale.

**68.** Procédé selon la revendication 67, dans lequel la composition à odeur réduite comprend en outre au moins un ingrédient supplémentaire choisi dans le groupe constitué par les biocides stabilisants, les parfums, les arômes, les colorants, et des mélanges de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1501873 A1

- WO 03095497 A

### Non-patent literature cited in the description

- **M.M. Bradford.** *Anal. Biochem.,* 1976, vol. 72, 248-254
- **Walter Noll's.** Chemistry and Technology of Silicones. Academic Press, 1968

- Volatile Silicone Fluids for Cosmetics. Cosmetics and Toiletries. Todd & Byers, January 1976, vol. 91, 27 32